# EUROPEAN PATENT APPLICATION

(11) **EP 4 763 977 A1**
(43) Date of publication of application: **24.06.2026**
(21) Application number: 24222592.8
(22) Date of filing: 20.12.2024
(51) Int. Cl.: C12N 5/0775, A61K 35/00, A61K 35/28

(54) **EXTRACELLULAR VESICLES**

(71) Applicant: Fairway Lab AG, 8045 Zürich (CH)
(72) Inventor: Janssens, Susan, 8045 Zürich (CH)
(74) Representative: Gill Jennings & Every LLP

(57) **Abstract**

Extracellular vesicles produced from genetically modified cells, which have been modified to increase the activity of at least PGC-1α and PGC1β, methods of manufacturing the same and constructs used in manufacture and medical uses thereof.

## Description

### FIELD OF THE INVENTION

The present invention provides extracellular vesicles enriched in whole mitochondria or mitochondrial biomolecules, cells which have been genetically modified to produce such extracellular vesicles, and nucleic acid constructs which may be used in the genetic modification.

### BACKGROUND

Ageing is a primary risk factor for neurodegenerative diseases, including dementia and Alzheimer disease (AD), Parkinson's disease (PD), motor neurone disease (MND) and multiple sclerosis (MS). Neurodegenerative diseases are becoming increasingly more widespread in the global population, particularly due to worldwide demographic aging and increasing lifespans. Due to aging playing a large factor in the onset of these conditions, diagnosis is most common in individuals over the age of 65. In 2021, 761 million people worldwide were aged 65 and older, which has been estimated to rise to 1.6 billion by 2050 (United Nations, 2023).

The effects of neurodegenerative disease on the population are both personal and economic. Most of these conditions are associated with a significantly decreased quality of life, with additional care often being required either through family members, caregivers, or care homes. The annual cost for neurodegenerative diseases in the U.S. amounted to $655 billion in 2020, a figure which includes both direct medical and non-medical costs and indirect costs from lost productivity and uncompensated caregiving hours (Partnership to Fight Chronic Disease, 2021).

The process of aging is multifactorial, with the key hallmarks having been identified as genomic instability, telomere attrition, epigenetic alterations, loss of proteostasis, deregulated nutrient sensing, mitochondrial dysfunction, cellular senescence, stem cell exhaustion, and altered intercellular communication (López-Otín et al. Cell 2013). These hallmarks are split into primary hallmarks, antagonistic hallmarks and integrative hallmarks. Primary hallmarks (genomic instability, telomere attrition and epigenetic alteration) are negative and damaging events that occur in the cell. Antagonistic hallmarks (deregulated nutrient sensing, mitochondrial dysfunction and cellular senescence) are compensatory responses designed to ameliorate the primary damage and are beneficial in low levels. However, at increased levels they too become harmful and can induce further damage. Integrative hallmarks (stem cell exhaustion and altered intercellular communication) arise resulting from the cumulative damage induced by the primary and antagonistic hallmarks and have direct effects on tissue homeostasis and function. In combination, these hallmarks are responsible for the functional decline associated with ageing.

Presently, there are very few therapies that offer any effectiveness in in treating the complex issues of the ageing brain caused by these various constituent elements. The brain is a vastly complex organ, and few neurodegenerative diseases are caused by a single defect. For example, AD is a multifactorial disease with multiple mechanisms implicated in its pathology, including AD pathology abnormal tau protein metabolism, β-amyloids, inflammatory responses, and cholinergic and free radical damage (Breijyah and Karaman, Molecules 2020).

Certain classes of small molecule drugs have been developed and approved to help manage the progression of AD. The first of these are acetylocholinesterase inhibitors (AChEls) (such as onepezil, galantamine and rivastigmine), which help increase the availability of the neurotransmitter acetylcholine at synapses, which is decreased both in concentration and function in patients with AD. The second of these are N-methyl d-aspartate (NMDA) antagonists (such as memantine), which assists in blocking NMDA-mediated calcium intake flux to help mitigate the harmful effects of elevated glutamate levels that can lead to neuronal dysfunction (Yiannopoulou and Papageorgiou, J Cent Nerv Syst Dis. 2020). These treatments are effective only in treating the symptoms of AD but fail to prevent or cure the disease.

Immunotherapy approaches have also been developed to help reduce the cognitive decline seen in Alzheimer's, with the monoclonal antibodies lecanemab and donanemab both targeting and facilitating the removal of Aβ plaques in the brain. Both have received approval by the FDA and MHRA, with lecanemab gaining approval from the two authorities in January 2023 and August 2024 respectively, and donanemab in July 2024 and October 2024 respectively.

However, due to the complexity of neurodegenerative disease pathologies, drugs and biologics developed to target a single mechanism are of limited efficacy in treating these conditions. Therefore, there is great desire to find a single agent, or a combination of agents, which can help tackle the multi-faceted nature of neurodegenerative disease pathology.

Cell therapy has emerged as a promising therapeutic modality due offering many advantages over conventional small molecules and other biologics. Since cells are living, they can perform and respond to many biological functions when implanted in biological settings and have been shown to facilitate activities such as regeneration, restoration of function, and attacking malignancies. These favourable effects have been harnessed in the development of therapies for many indications, and this therapeutic field represents a promising avenue to continue to help treat many medical conditions.

Over recent years, a cell-derived structure has proven of great interest as a potential therapeutic agent, drug delivery system, and disease biomarker. Extracellular vesicles (EVs) are biologically active lipid-bound particles that are naturally released from all cells. EVs vary in their composition depending on the cell they derive from, but typical cargoes include proteins (e.g. growth factors, extracellular matrix components, cytoskeletons and metabolic enzymes), lipids (e.g. cholesterol and ceramides), nucleic acids, metabolites, and organelles. Due to their ability mediate intercellular communication and molecular transfer, EVs facilitate a vast number of functions, and play roles in cytokine transport, antigen presentation to T cells, lipid transport, antioxidant transport, angiogenesis, and transferring mRNAfor translation to a recipient cell (van Niel et al. Nature Reviews Molecular Cell Biology 2018, Sun and Chang, Tzu Chi Med J. 2024).

Early research into the physiological effects of EVs identified their potential in regenerative medicine, wherein exosomes released by mesenchymal stem cells (MSCs) were shown to be the cardioprotective factor in MSC paracrine secretion (Lai et al. 2010). Due to their ability to rapidly deliver functional proteins and genetic material into recipient cells, EVs represent an ideal mechanism and vehicle to stimulate the repair and recovery of damaged cells and tissues.

In comparison to cell-based therapies, EVs offer a number of advantages, including being less immunogenic due to a lower abundance of membrane proteins such as MHC complexes, longevity when being transported and stored, and less risk of tumour generation due to being unable to replicate after injection (Murphy et al. Experimental & Molecular Medicine 2019).

EVs derived from stem cells have been of particular interest due to their ability to mimic the effects of highly potent cells and perform powerful functions such as modulating immune pathways, promoting effector cell migration and proliferation, and reducing apoptosis (Kou et al. Cell Death & Disease 2022). EVs derived from mesenchymal stem cells (MSCs) have been shown to impart neuro-specific benefits. N2A neuroblastoma cells exposed to MSC-EVs reduced both secreted and intracellular Aβ peptide levels (Katsuda et al. Scientific Reports 2013). A similar effect was shown in MSC and rat neuron co-culture exposed to soluble Aβ peptide oligomers, which could mediate the clearance of the Aβ peptides through internalisation (de Godoy et al. Journal of Biological Chemistry 2018). Additional effects observed have included increased neurogenesis, neural plasticity and functional recovery after stroke and promoting the mobility and angiogenesis of brain microvascular endothelial cells after oxygen-glucose deprivation (Yin et al. Biomarker Research 2019). EVs have also been shown to assist in reversing typical aging markers.

Various EV engineering strategies have been previously developed to increase their therapeutic potential. These approaches have been directed towards improving their circulation time so as to ensure efficient delivery to target organs, and the loading of therapeutic agents into EVs specifically tailored to treat certain diseases. Targeting strategies include chemical modification to the surface of EVs by biomolecules such as peptides and antibodies to direct them to a cell-surface target. Additionally, genetic modification of the donor cells has been used to introduce cell-surface peptides onto EVs to allow targeting in a similar way. Additionally, synthetic protein systems have been used to capture and release proteins from EVs using optically reversible protein-protein interactions, and overexpression of target proteins has also been used to help capture the desired molecules for delivery (Dayem et al. Nano Convergence, 2024). For example, overexpression of stromal cell-derived factor 1 (SDF-1), which helps facilitate the attraction of stem cells, in MSCs was shown to aid cardiac repair post-myocardial infarction (Gong et al. Journal of Cellular Physiology 2019). Additionally, overexpression of the macrophage migration inhibitory factor (MIF) in MSCs showed enhanced myocardial repair by preventing early inflammation at the damaged site (Zhang et al. Aging 2019).

The process of aging and neurodegeneration is multifactorial, and it is therefore challenging to develop effective therapeutic strategies. There exists a continuing need for the provision of therapeutically effective biologics to help treat and manage the progression of neurodegenerative diseases.

### SUMMARY OF INVENTION

The present invention is based on the inventor's revelation that EVs that have been engineered to comprise an increased density of mitochondrial biomolecules may provide an effective treatment for neurodegenerative diseases and that such EVs may be manufactured by expressing or overexpressing certain genes in suitable stem cells, such as MSCs.

Accordingly, in a first aspect of the invention, there is provided an isolated extracellular vesicle derived from a genetically modified cell, wherein the cell is engineered to express at least two exogenous genes encoding at least two proteins of interest or to overexpress at least two native genes encoding at least two proteins of interest, wherein the at least two proteins of interest comprise PGC-1α and PGC-1β.

The extracellular vesicle may comprise an increased density of whole mitochondria or mitochondrial biomolecules compared to that of an extracellular vesicle produced from an equivalent cell, wherein the equivalent cell has not been engineered to express the at least two exogenous genes encoding at the least two proteins of interest, or wherein the equivalent cell has not been engineered to overexpress the at least two native genes encoding the at least two proteins of interest.

Further genes that encode further proteins of interest may be selected to be expressed as exogenous genes or to be overexpressed as native genes in the cell. In an embodiment, additional proteins of interest are selected from the group comprising: PPAR, NRF2, NRF1, TFAM, DRP1, OPAC1, SIRT3, POLG, TOP1MT, MRPL12, MRPS16, SIRT1, AMPK, FOXO3, KLOTHO, NeuroD1, BDNF, REST, and TERT. Preferably, the additional protein of interest is TERT, PPAR, NRF1 or NRF2. Preferably, the TERT protein is hTERT. Preferably, the PPAR protein is PPARγ.

In a second aspect of the invention, there is provided a method of preparing an isolated extracellular vesicle, the method comprising:
(a) subjecting a cell to genetic modification such that the cell is engineered to express at least two exogenous genes encoding at least two proteins of interest or to overexpress at least two native genes encoding at least two proteins of interest, wherein the at least two proteins of interest comprise PGC-1α and PGC-1β;
(b) culturing the cell, such that the extracellular vesicle is released from the cell; and
(c) isolating the extracellular vesicle released from the cell.

In a third aspect of the invention, there is provided a genetically modified cell, wherein the cell is engineered to express at least two exogenous genes encoding at least two proteins of interest or to overexpress at least two native genes encoding at least two proteins of interest, wherein the at least two proteins of interest comprise PGC-1α and PGC-1β.

In a fourth aspect of the invention, there is provided a method of increasing the density of whole mitochondria or mitochondrial biomolecules in a cell, the method comprising subjecting the cell to genetic modification such that the cell is engineered to express at least two exogenous genes encoding at least two proteins of interest or to overexpress at least two native genes encoding at least two proteins of interest, wherein the at least two proteins of interest comprise PGC-1α and PGC-1β.

In a fifth aspect of the invention, there is provided a nucleic acid construct comprising a nucleic acid sequence comprising coding sequences for at least two genes which encode at least two proteins of interest, wherein the at least two proteins of interest comprise PGC-1α and PGC-1β.

In a sixth aspect of the invention, there is provided a host cell comprising the nucleic acid according to the fifth aspect of the invention.

In a seventh aspect of the invention, there is provided a cell-free expression system comprising the nucleic acid according to the fifth aspect of the invention.

In an eighth aspect of the invention, there is provided a pharmaceutical composition comprising the isolated extracellular vesicle according to the first aspect of the invention, the genetically modified cell according to the third aspect of the invention, the nucleic acid construct according to the fifth aspect of the invention, the host cell according to the sixth aspect of the invention, or the cell-free expression system according to the seventh aspect of the invention.

In a ninth aspect of the invention, there is provided a kit comprising the isolated extracellular vesicle according to the first aspect of the invention, the genetically modified cell according to the third aspect of the invention, the nucleic acid construct according to the fifth aspect of the invention, the host cell according to the sixth aspect of the invention, the cell-free expression system according to the seventh aspect of the invention, or the pharmaceutical composition according to the eighth aspect of the invention.

In a tenth aspect of the invention, there is provided the isolated extracellular vesicle according to the first aspect of the invention, the genetically modified cell according to the third aspect of the invention, the nucleic acid construct according to the fifth aspect of the invention, the host cell according to the sixth aspect of the invention, the cell-free expression system according to the seventh aspect of the invention, the pharmaceutical composition according to the eighth aspect of the invention, or the kit according to the ninth aspect of the invention, for use in medicine.

### BRIEF DESCRIPTION OF DRAWINGS

Figure 1 shows an experimental overview of the production, isolation, and application of extracellular vesicles of the invention.
Figure 2 shows an experimental overview of the delivery of genes encoding proteins of interest to a cell through a vector via liposome transfection, and confirmation via PCR.
Figure 3 shows vector constructs comprising genes encoding proteins of interest of the invention as part of a transposable element.
Figure 4 shows vector constructs comprising the PiggyBac transposase which may be used to insert transposable elements using the PiggyBac transposon system.
Figure 5 shows the relative fold gene expression of genes encoding hTERT, PGC-1α and PGC-1β in umbilical cord-derived mesenchymal stem cells (ucMSCs) through real-time quantitative PCR (qPCR) using PPIA (Peptidylprolyl Isomerase A) as a housekeeping gene calculated by the 2-delta-deltaCt (2^{-ΔΔCt}) method, measured for different liposome and plasmid ratios.
Figure 6 shows a workflow comprising various markers which may be used to screen cells for senescence.
Figure 7 shows a protocol for inducing DNA damage in neural progenitor cells (NPCs) with doxorubicin.
Figure 8 shows a protocol for inducing DNA damage in human microglial cells (HMC3 cells) with doxorubicin.
Figure 9 shows a protocol for inducing DNA damage in rat hippocampal cells with doxorubicin.

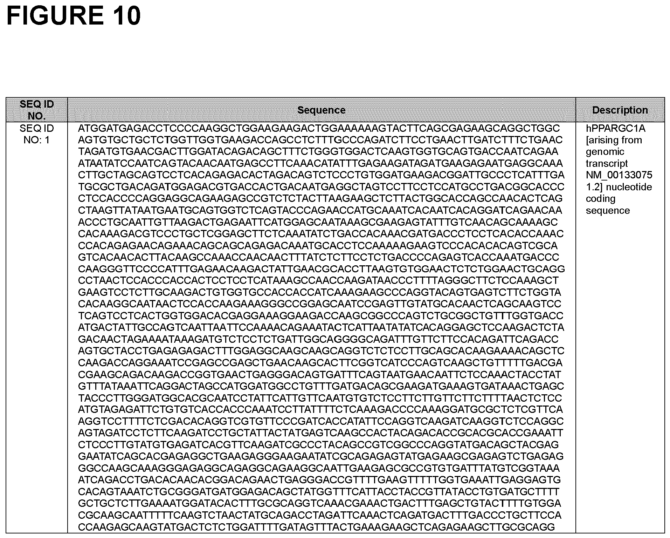

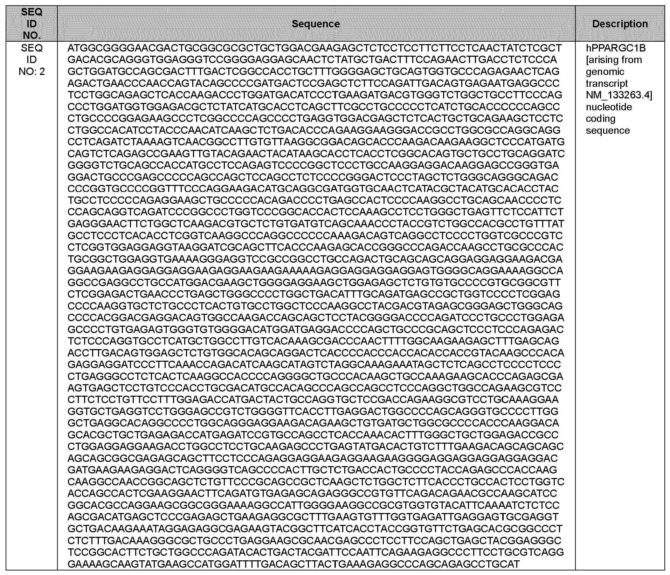

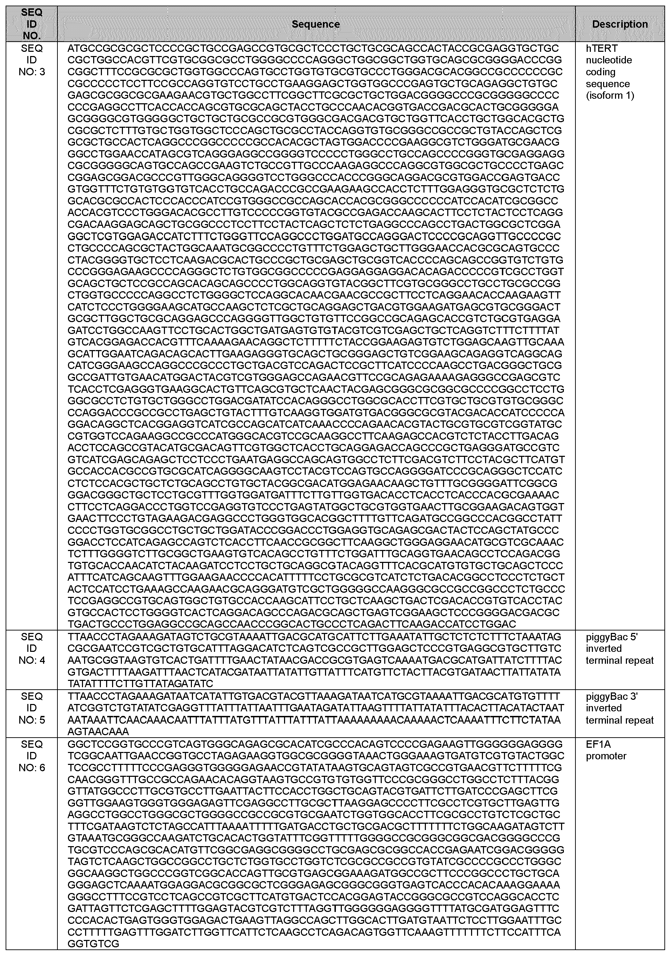

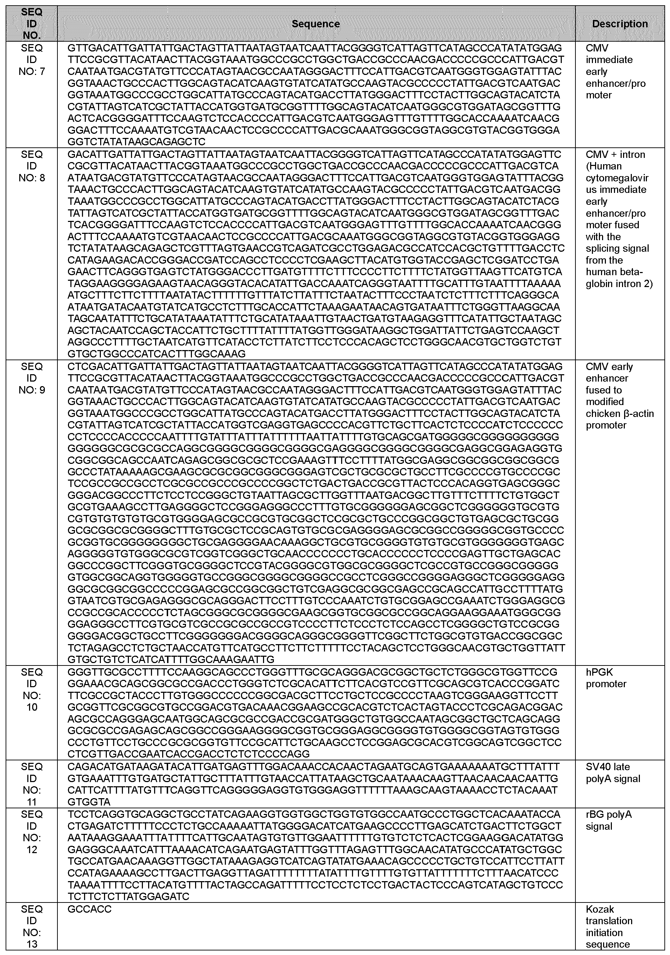

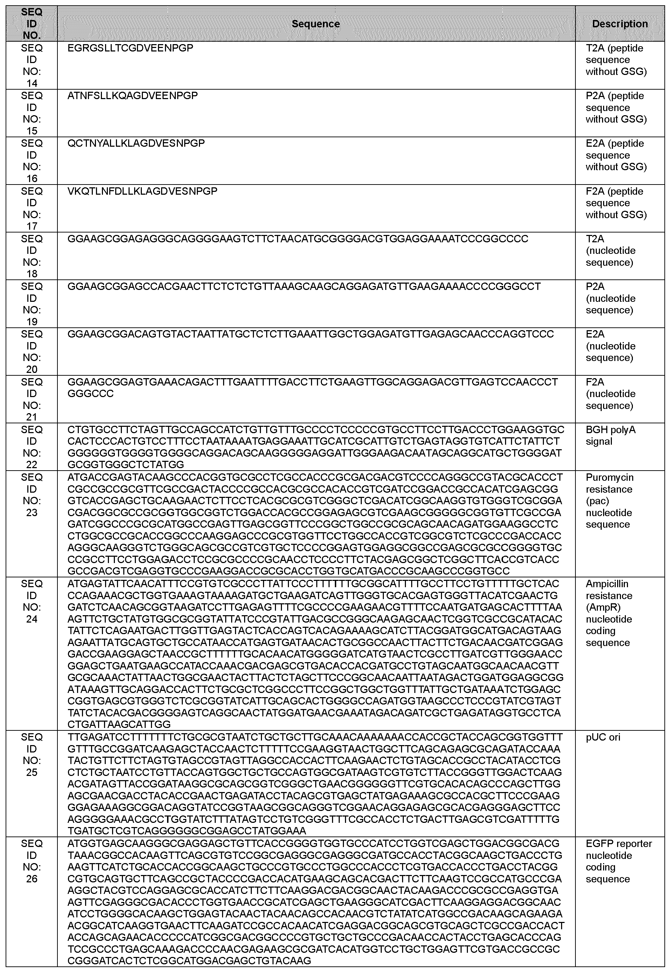

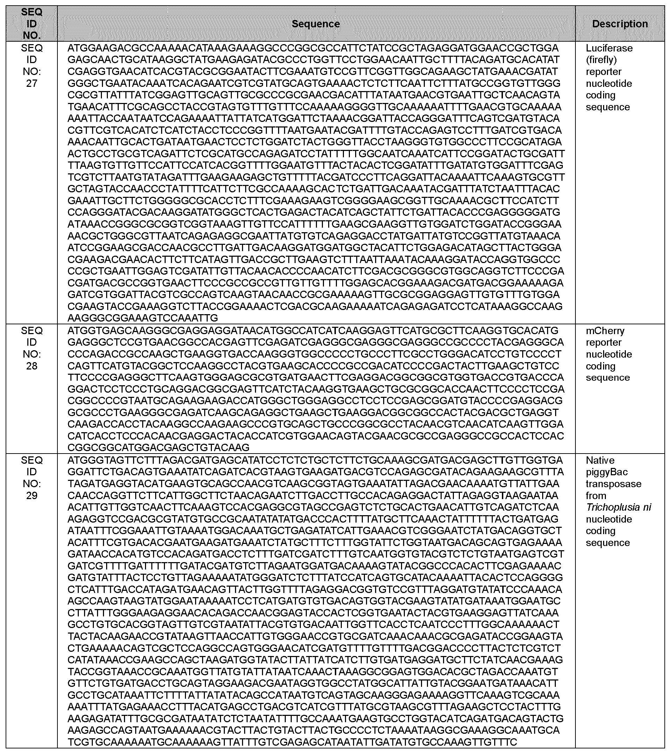

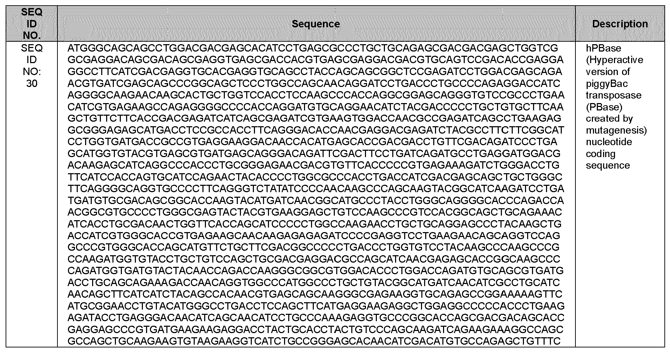

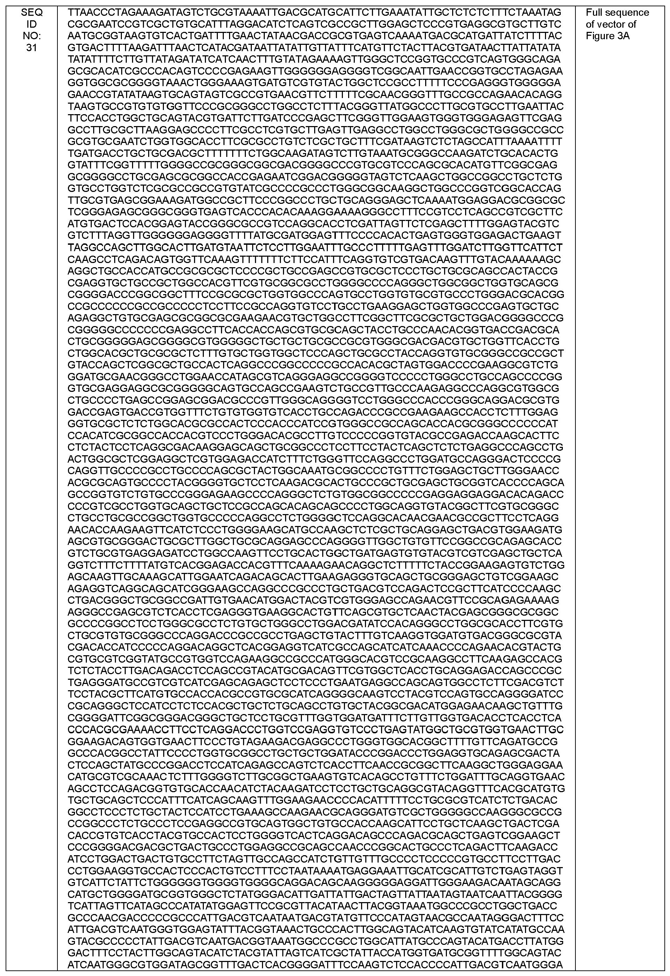

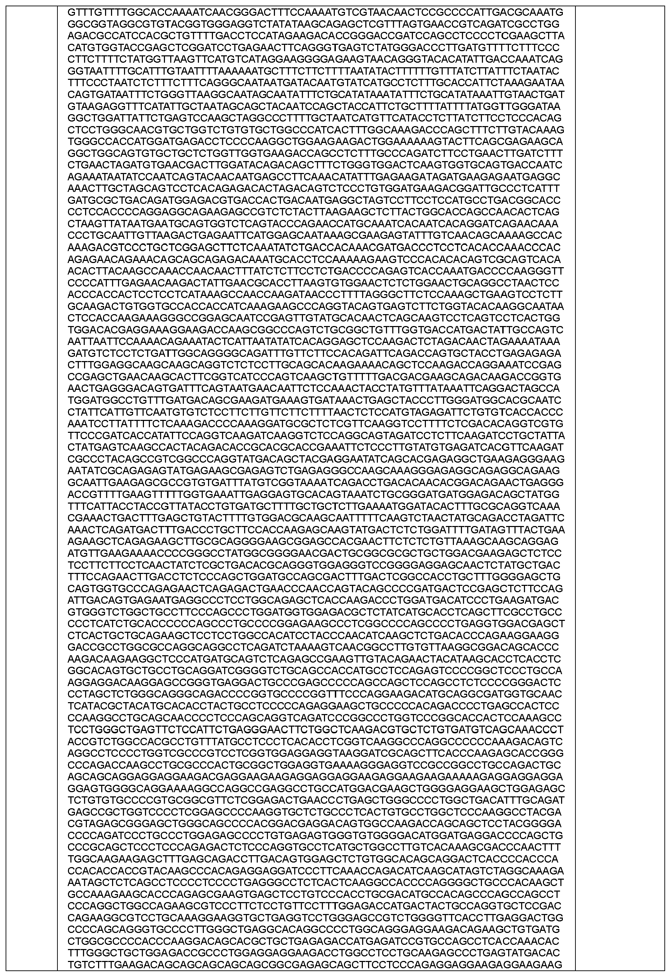

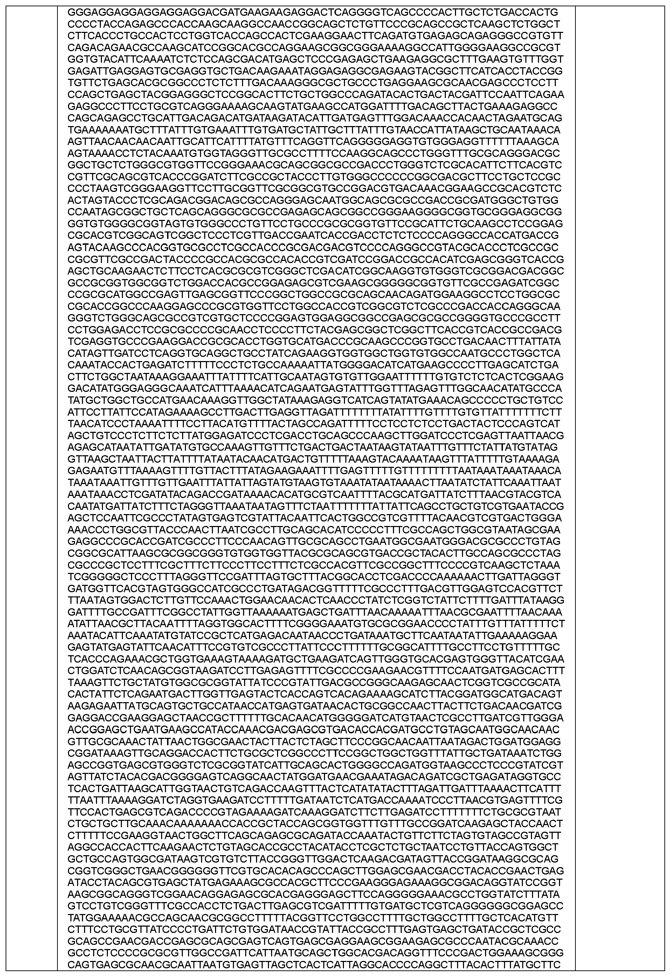

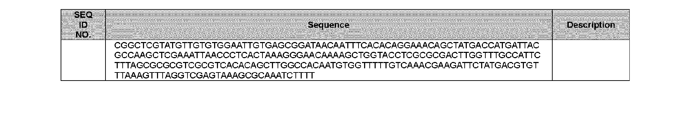

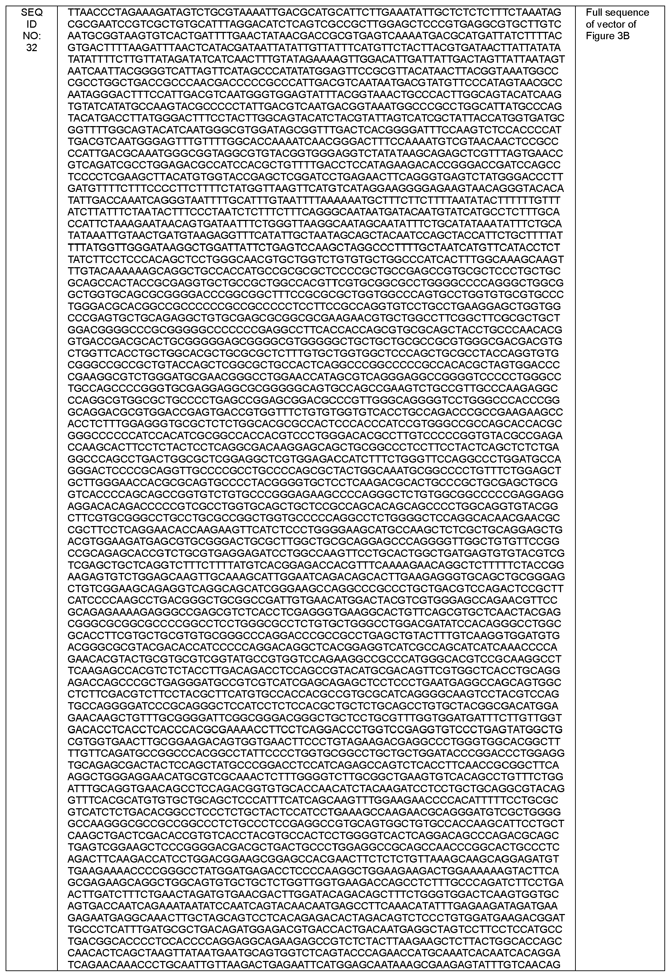

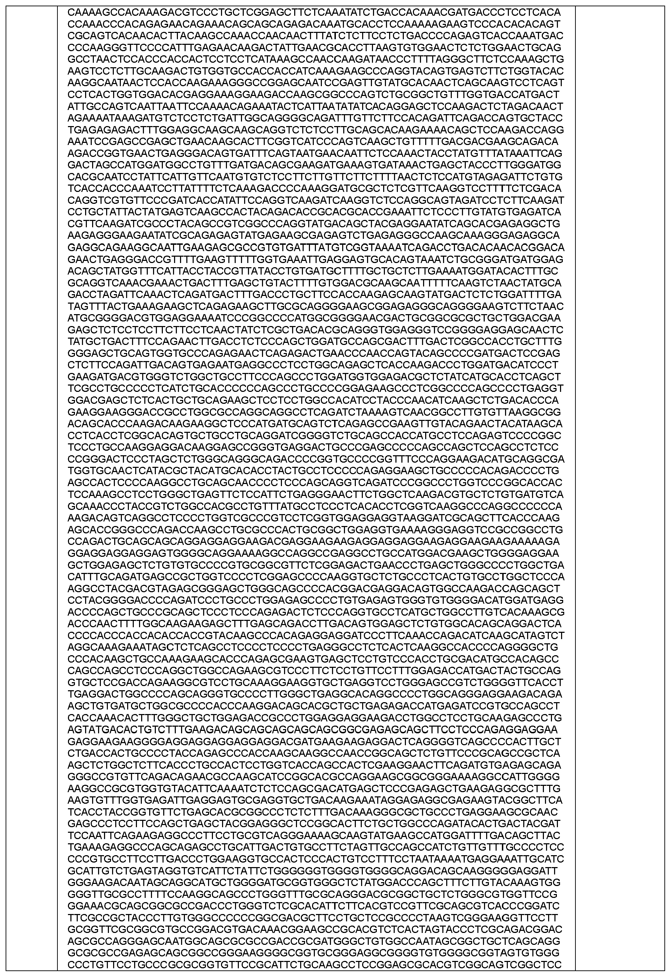

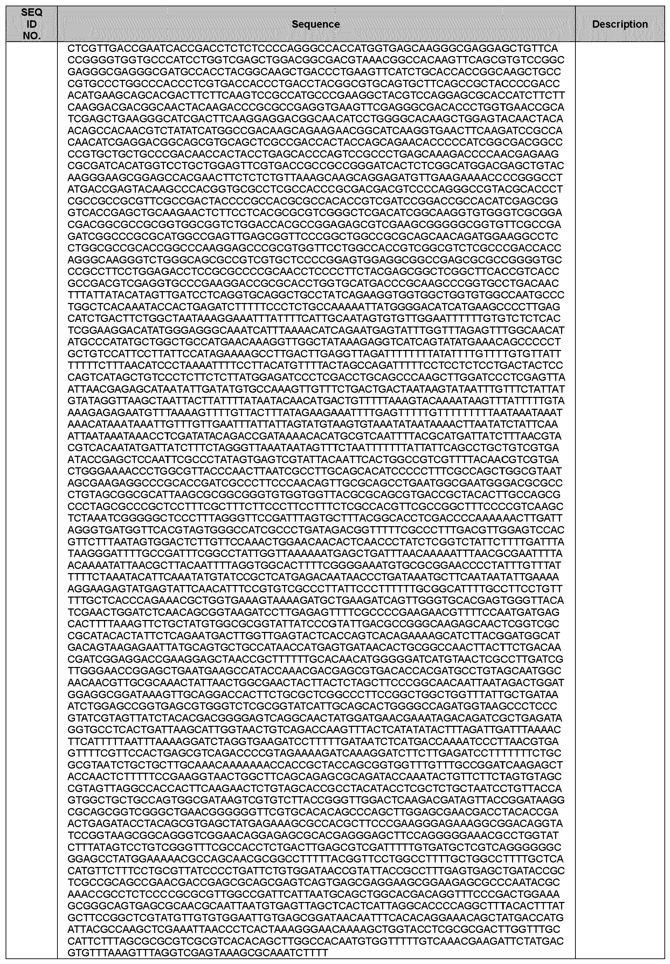

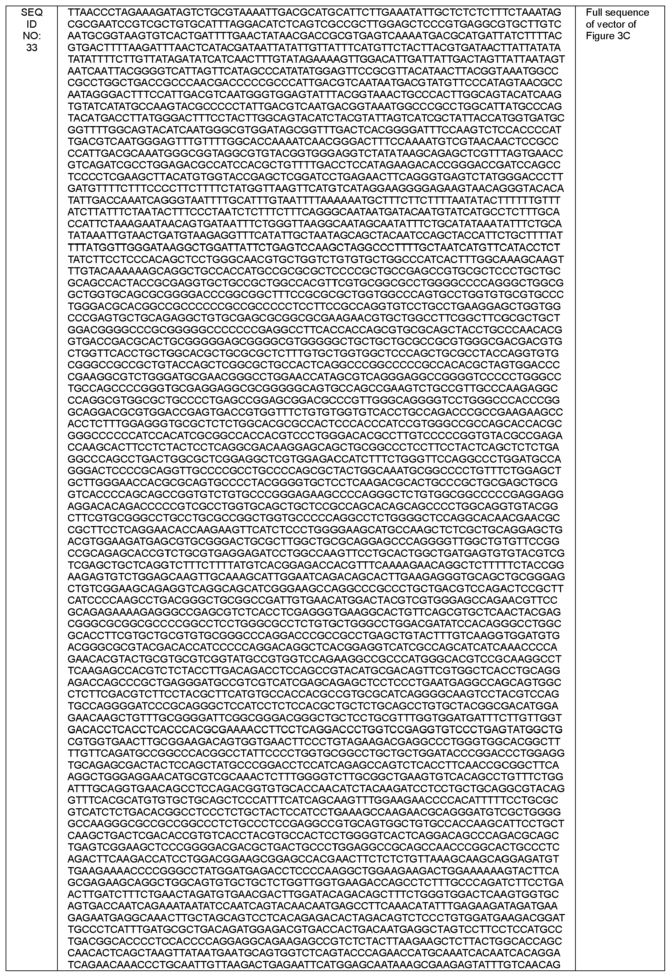

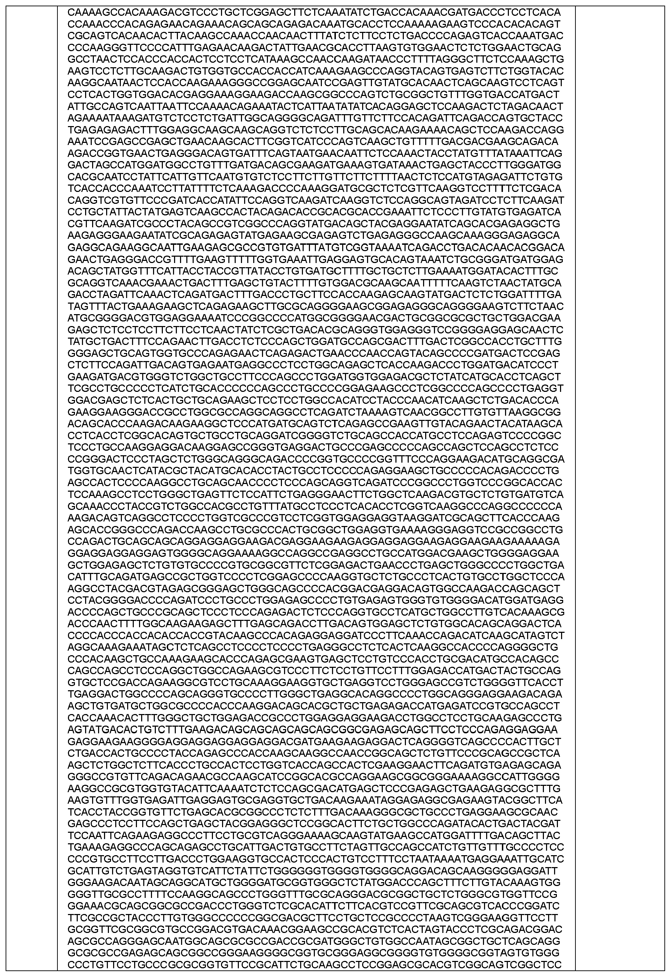

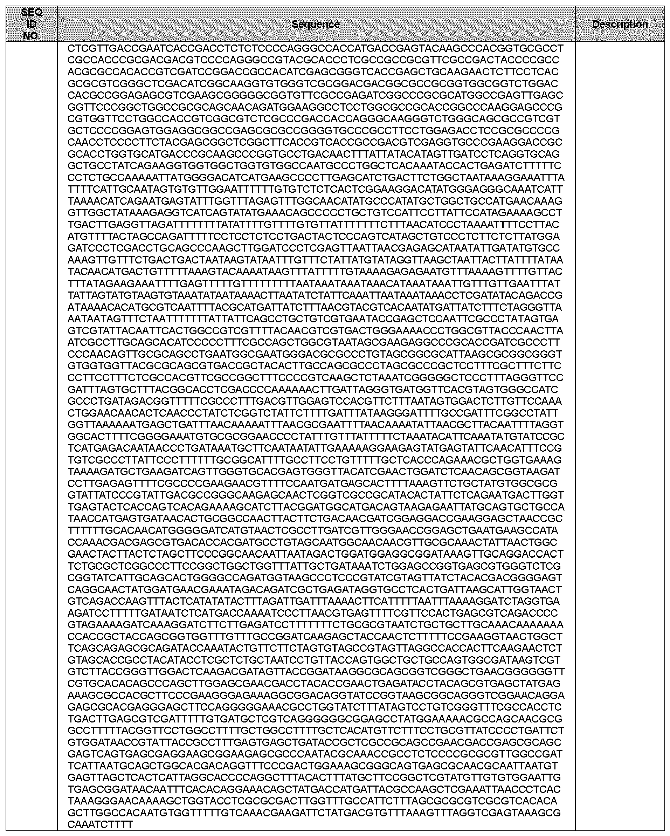

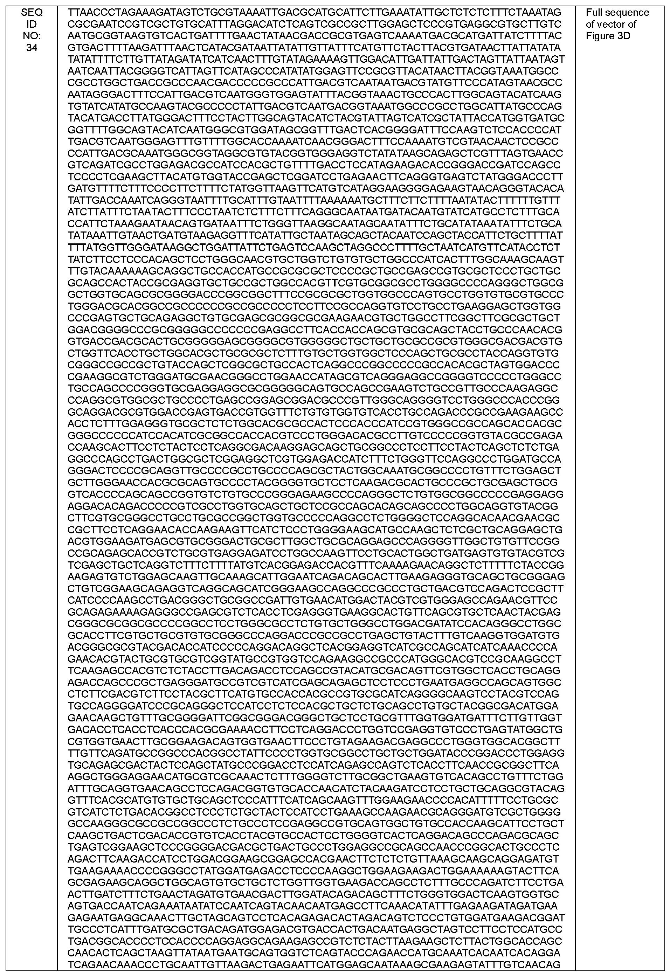

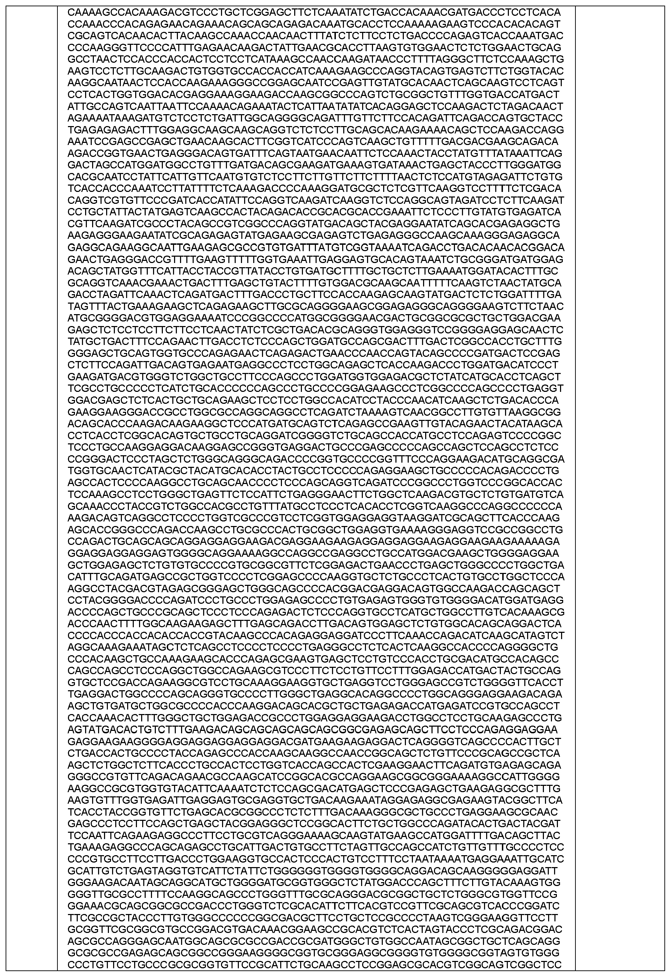

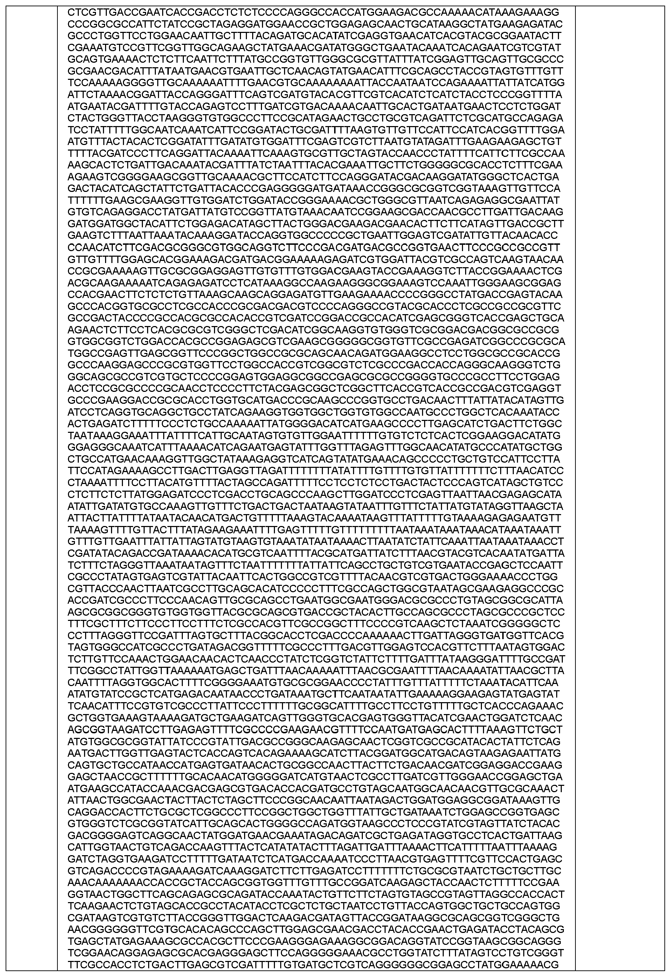

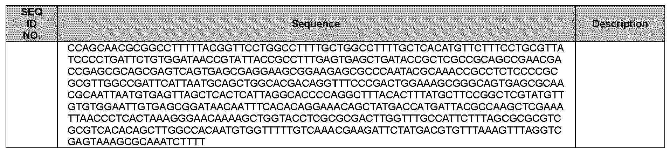

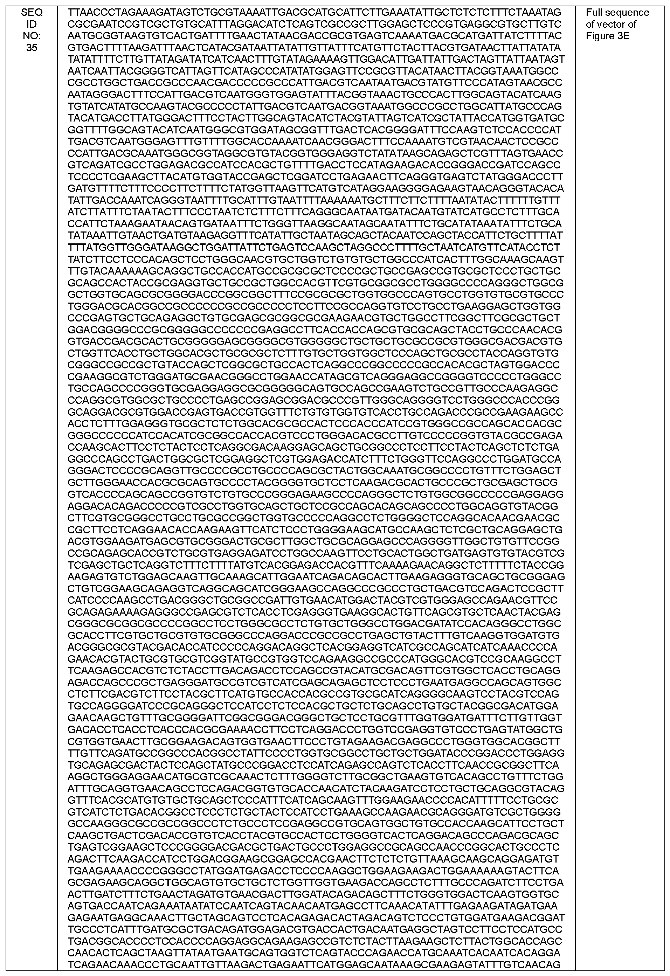

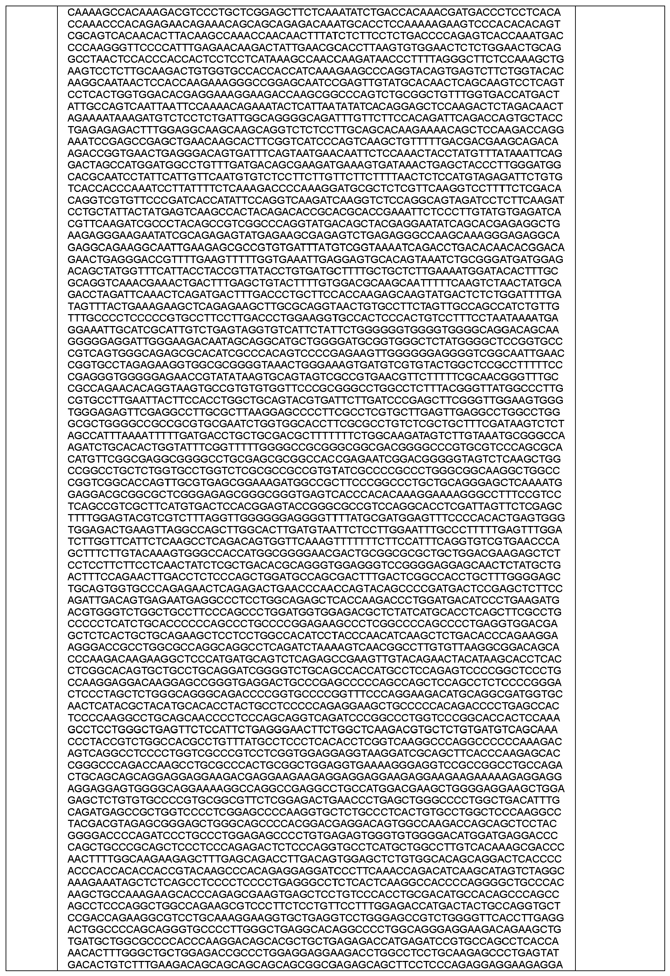

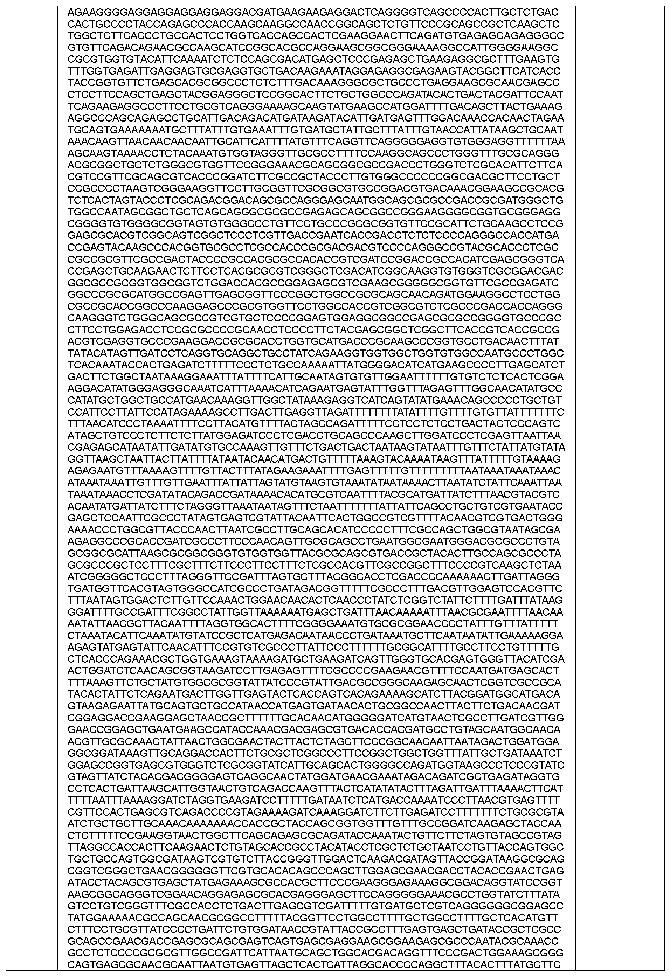

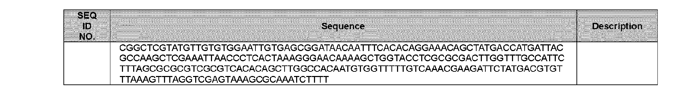

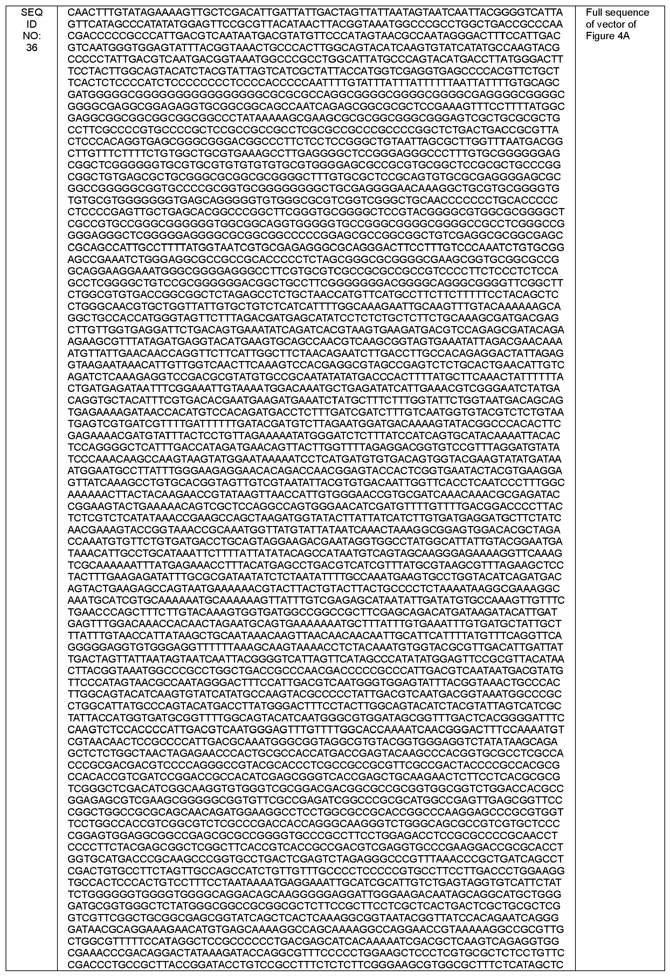

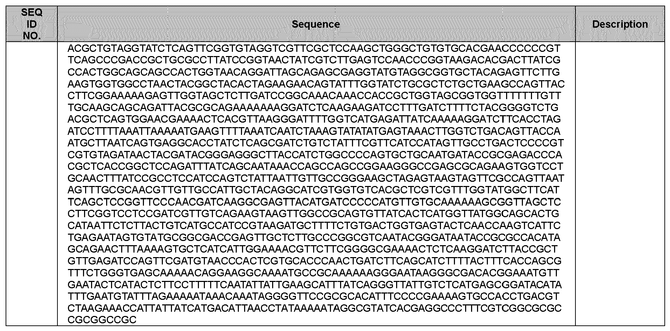

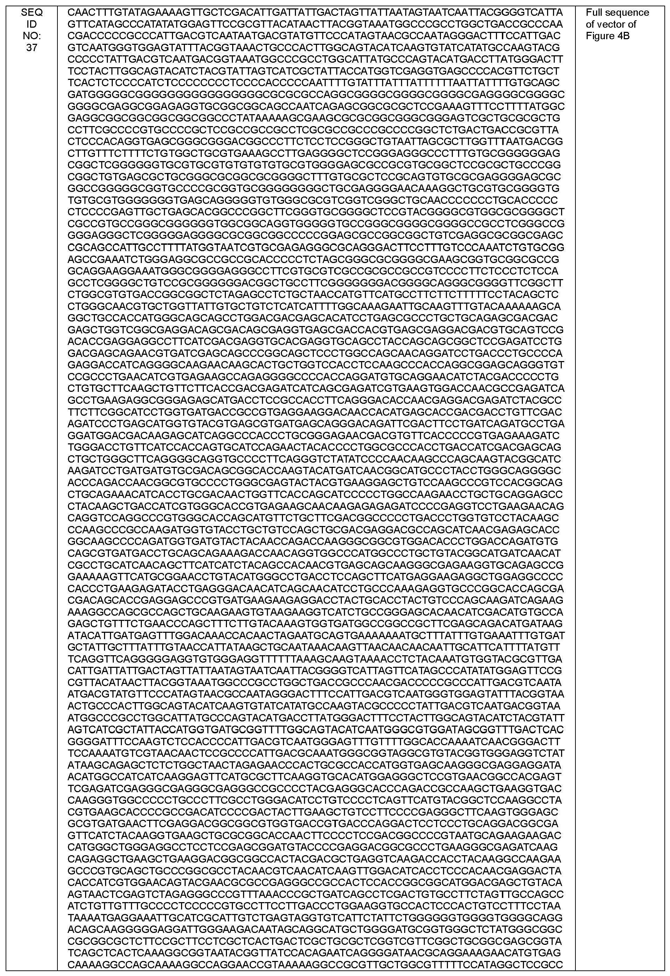

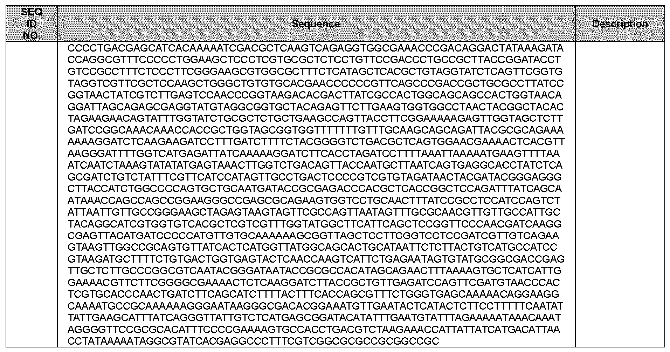

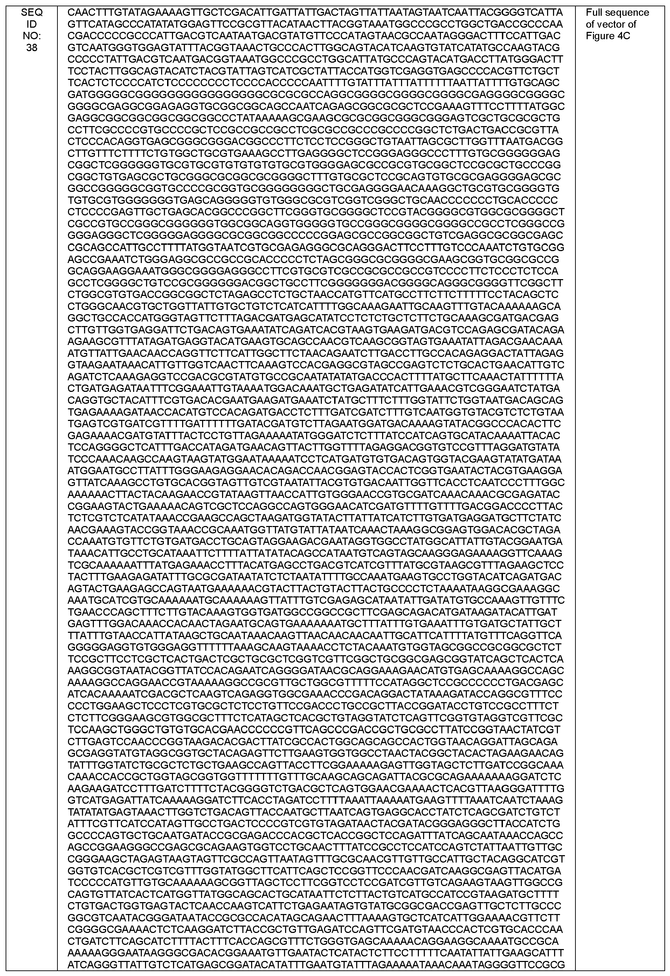

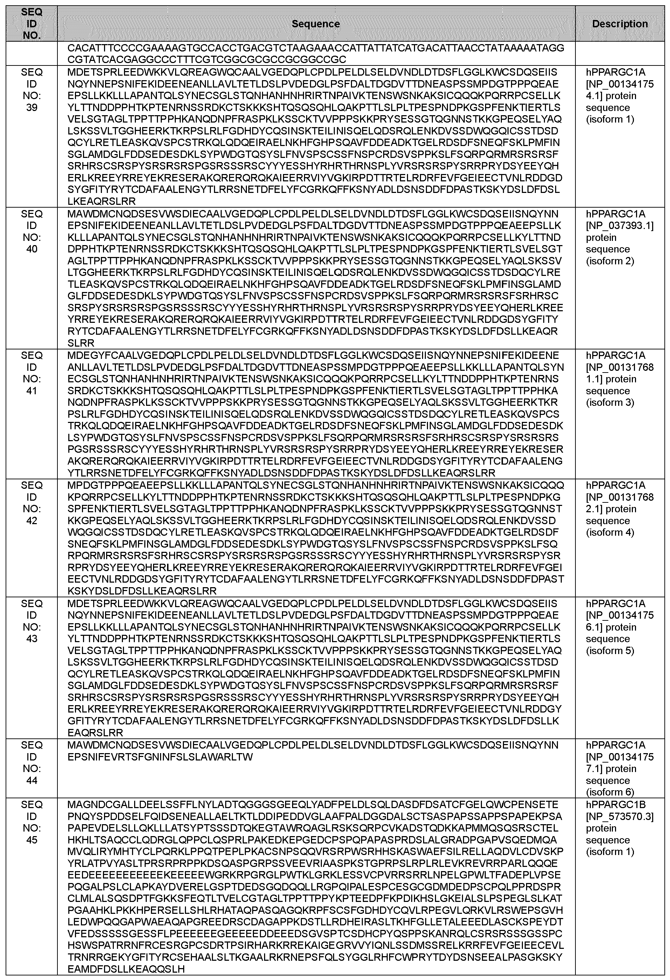

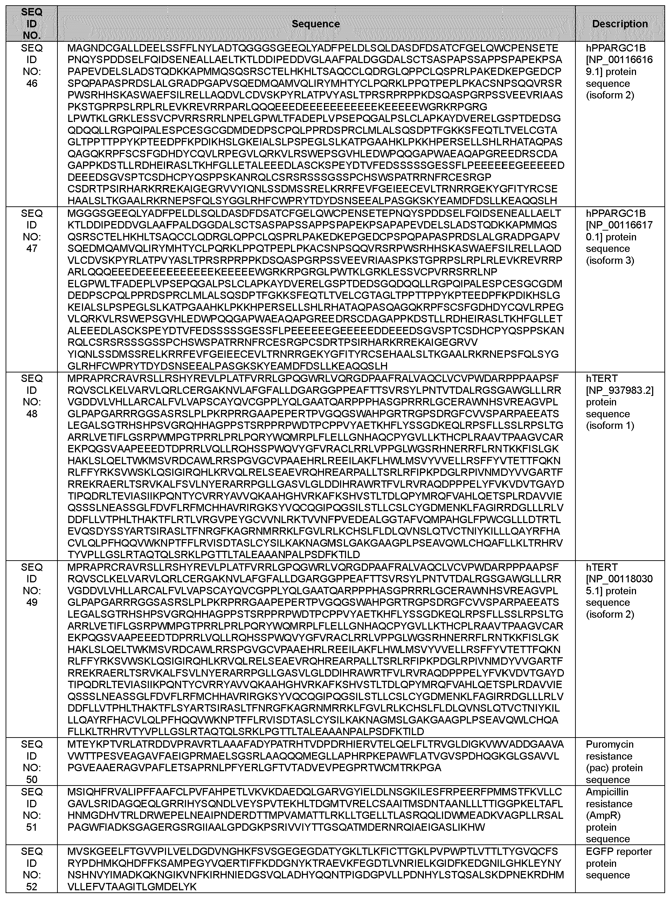

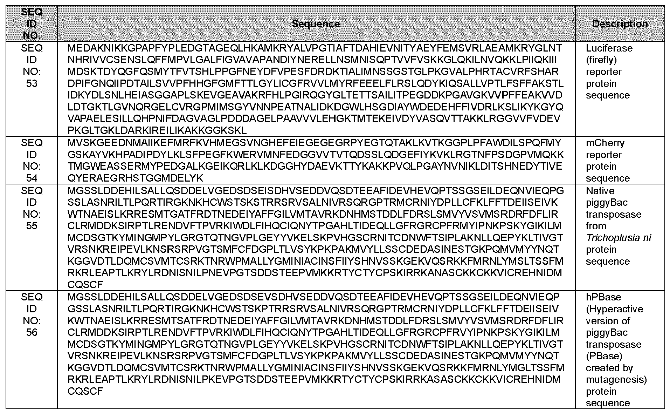

Figure 10 shows a table which comprises sequences of the invention.

### DETAILED DESCRIPTION

Unless defined otherwise, the scientific and technical terms used herein should be taken to have the same meaning as would be understood by a person of ordinary skill in the art. Therefore, they should be taken to have their natural meaning unless indicated otherwise. Relevant definitions provided herein, which are intended to take precedent over any external definition in the circumstance that any meaning and/or scope of terms indicated herein is not clear.

As used herein, unless indicated otherwise, the term "comprise", "comprises" and "comprising" are understood to reference the inclusion of a stated feature or group of features without excluding any other feature or group of features.

The exemplary methods and materials disclosed herein shall not limit this disclosure, and a person of ordinary skill in the art will understand how to adapt, or use equivalents to, the methods and materials disclosed herein to practice or test any of the embodiments of this disclosure.

Any specific embodiments disclosed by a description are not intended to be exhaustive or to limit the disclosure to such specific embodiments. A person of ordinary skill in the art will understand relevant modifications which can be made to any of the specific embodiments disclosed herein and therefore should take any specific embodiments simply as illustrative teachings of the present invention.

The present invention provides various products and methods which comprise EVs isolated from genetically modified cells, which may comprise an increased density of mitochondrial biomolecules compared to those isolated from a non-genetically modified cell, and production methods thereof.

The present invention relates in part to extracellular vesicles. As used herein, the term "extracellular vesicles" relates to lipid bilayer-delimited particles that cells release into their surroundings and can't replicate. They encapsulate a variety of bioactive molecules which play crucial roles in cell-to-cell communication in both pathological and physiological contexts. Many different types of extracellular vesicles have been identified, but the two main subtypes are small extracellular vesicles (sEVs) and microvesicles (mVs). sEVs, which include exosomes, are created during the endocytosis pathway through the inwards budding of the endosome and are subsequently released through fusion with the cell membrane, and usually have a diameter of 30 nm to 150 nm. mVs have a larger diameter between 100 nm and 1000 nm and originate from the outwards budding of the cytoplasmic membrane.

The invention further relates in part to genetically modified cells. Such cells have been genetically modified to increase the expression of certain genes which encode proteins of interest, either by being engineered to express exogenous genes encoding proteins of interest or overexpress native genes encoding proteins of interest, or both. This will result in the increase of expression products (i.e. the proteins of interest) produced from the increased transcription and translation of the genes encoding the proteins of interest. An increased density of these expression products will therefore exist in the genetically modified cell, and therefore will be incorporated into extracellular vesicles derived from the genetically modified cell, which may include these expression products at a higher density. Furthermore, the enhanced expression of particular genes encoding proteins of interest may also stimulate the biogenesis of whole mitochondria and increase the level of mitochondrial biomolecules in the genetically modified cell, such that an increased density of whole mitochondria or mitochondrial biomolecules may be present in the cell. It is expected that this will similarly result in an increased density of whole mitochondria or mitochondrial biomolecules within extracellular vesicles derived from the genetically modified cells.

The invention further relates in part to nucleic acid constructs to produce these genetically modified cells, which comprise the necessary genes encoding the proteins of interest to produce extracellular vesicles with an increased density of whole mitochondria or mitochondrial biomolecules compared to that of an extracellular vesicle produced from an equivalent cell which has not been subject to such engineering.

The invention further relates in part to methods to produce the extracellular vesicles comprising an increased density of whole mitochondria or mitochondrial biomolecules, and methods to produce the genetically modified cells from which those vesicles are derived.

Additionally, various pharmaceutical compositions comprising the products of the invention, and medical uses for such pharmaceutical compositions and products, are provided.

It should be understood that certain features of the present invention may be readily applied to various embodiments (for example, regarding the nucleic acid constructs, cells, extracellular vesicles, or methods comprising the same), and that certain method steps of one aspect of the invention will also be applicable to other aspects of the invention. The skilled person will understand where this applies, and therefore be able to make relevant modifications to allow the practicing of the full scope of the invention as disclosed herein.

In a first aspect of the invention, there is provided an isolated extracellular vesicle derived from a genetically modified cell, wherein the cell is engineered to express at least two exogenous genes encoding at least two proteins of interest or to overexpress at least two native genes encoding at least two proteins of interest, wherein the at least two proteins of interest comprise PGC-1α and PGC-1β.

It is envisaged that the extracellular vesicle will comprise an increased density of whole mitochondria or mitochondrial biomolecules compared to that of an extracellular vesicle produced from an equivalent cell, wherein the equivalent cell has not been engineered to express the at least two exogenous genes encoding the at least two proteins of interest, or wherein the equivalent cell has not been engineered to overexpress the at least two native genes encoding the at least two proteins of interest.

The present invention is predicated on the view that increasing the expression of genes encoding particular proteins of interest in a donor cell may produce EVs with enhanced therapeutic utility compared to those derived from an equivalent cell. In particular, the inventors have identified that PGC-1α and PGC-1β, involved in the mitochondrial biogenesis pathway, may result in EVs with an increased density of mitochondrial biomolecules compared to those isolated from an equivalent cell, which may provide therapeutic benefits through the transfer of these elements (RNA, mRNA, proteins) to a recipient cell.

The EVs, cells, methods and pharmaceutical compositions provided by the present invention may offer several advantages over similar products and methods of the prior art.

One such advantage is that EV, due to their non-replicating nature come with various advantages over cell therapy including lesser risk of complication such as tumour formation, unwanted differentiation, or immune rejection. EVs are flexible in terms of their administration routes, and can circulate freely, unlike cell therapy which requires a localised injection. Furthermore, EVs are not limited by the blood brain barrier so can therefore target regions of the brain.

A further advantage is that the production of EVs of the invention has required the development of a stable cell line from which the EVs of the present invention are derived. The genetic modification of a donor cell to increase the expression level of PGC-1α and PGC-1β may enable the donor cell to produce whole mitochondria or mitochondrial biomolecules at an increased level compared to that of an equivalent cell, wherein these products may be incorporated into EVs produced from the donor cell. Upon generation of such a cell line, the production and isolation of the EVs of the invention would therefore become a significantly simpler task. Previous methods have used pharmacological modification of cells, such as the use of resveratrol, which is a non-permanent method and requires additional treatment steps and media supplementation.

A further advantage is that the EVs of the present invention may help impart increased anti-ageing properties upon cells and tissues to which they are administered. If such EVs are enriched in whole mitochondria or mitochondrial biomolecules, this may enhance similar effects exhibited by previous EVs.

As used herein, the term "derived" in the context of EVs relates to the cell of origin from which the EVs are released i.e. the EVs are produced from and comprise cellular components of the cell of origin, and are subsequently released into the extracellular space. For example, EVs released by mesenchymal stem cells (MSCs) may be described as "EVs derived from MSCs" or "MSC-derived EVs". Cells from which EVs are derived from may be referred to as "donor cells" or "parent cells", and cells which receive EVs (i.e. by membrane fusion or endocytosis) may be referred to as "recipient cells". EVs will be understood to contain cytosolic and membrane components from the cell from which they are derived.

As used herein, the terms "genetic modification" and "engineering" in the context of genetically modified cells, relates to the to the editing or manipulation of genomic, extrachromosomal or episomal DNA (including plasmids, mitochondrial DNA, and synthetic DNA constructs) carried out by any molecular biological method that would be familiar to the skilled person. The use of genetic modification to increase the expression level of PGC-1α and PGC-1β may ensure the precise control of the expression levels of these two desired genes while avoiding the consequences of off-target effects often associated with drug administration.

As used herein, the term "exogenous" in the context of genes relates to any nucleic acid that originates outside of an organism or cell of concern, i.e. a gene that is transfected into a cell from an external source. The cell may or may not encode and/or express the particular gene naturally, but the exogenous gene is one that is artificially transplanted into the cell to either provide the cell with a new copy of that gene or to enable overexpression or indeed just expression of that gene in the cell where the cell either expresses lower levels of the gene or does not express that gene at all.

As used herein, the term "protein of interest" relates to any protein which has been chosen to be used or analysed. Specific proteins of interest are outlined herein. Genes encoding the proteins of interest as defined herein are used in many embodiments of the present invention and would be understood by the skilled person.

Regarding the genetic modification of the cells concerned in the present invention, two general types of genetic modifications are envisaged. The first is a modification to facilitate the expression of exogenous genes which encode the proteins of interest. The second is a modification to facilitate the overexpression of native genes which encode the proteins of interest. These two methods could also be used together, as would be understood by a person of skill in the art. As used herein, "genetic modification" relates to the process of altering the genetic material of a cell, or genome of an organism, often to induce change in certain functions, characteristics or traits. The skilled person will understand that both these methods will result in the increased expression level of the genes which encode the proteins of interest (and therefore increased expression products derived from the transcription and translation of the genes encoding the proteins of interest) through the introduction of exogenous constructs comprising the genes encoding the proteins of interest (i.e. increasing the gene copy number and providing the cell with a gene that is readily accessible to express) or by reprogramming the usual expression pattern of native genes encoding the proteins of interest to increase their level of transcription. As used herein, the term "expression level" relates to a measured amount of expression activity of a particular gene. As used herein, the term "expression products" relates to molecules which have been derived from the transcription (and optionally their subsequent translation) of a gene, which includes RNA (including mRNA, miRNA, siRNA, ncRNA, IncRNA, eRNA, tRNA, and rRNA) and protein. For example, the expression product of a gene encoding a protein of interest will comprise the protein of interest.

Many methods of increasing the expression level of a gene (which may encode a protein of interest according to the invention) exist and will be familiar to the skilled person. Increased expression may be facilitated by plasmid-based methods, whereby a gene is introduced into the cell or organism of interest with a strong promoter to drive expression. Viral vector-based methods utilise genes cloned into their viral genome to then integrate into the genome of a host cell for expression, usually resulting in stable expression rather than transient expression, as with plasmid-based methods. Increased expression may also be achieved by introducing mRNA encoding a protein of interest into an expression system, offering a low-risk but short-lived differing expression profile. The CRISPR-endonuclease system may also be used to genetically modify a cell to increase the expression level of a gene, for example by using in conjunction with a ssDNA donor template so as to generate a double-stranded break in the genome to which homology-directed repair mechanisms may insert the gene comprised within the ssDNA donor template.

For the avoidance of doubt, it is contemplated that in an embodiment of the invention, at least one of the genes encoding a protein of interest may be endogenous to the cell and at least one of the genes encoding a protein of interest may be native to the cell and may or may not require overexpression to produce the EVs of the invention. Thus, the techniques provided herein may be blended as needed depending on the cell in question, its expression levels of the proteins of interest and its amenability to exogenous gene expression, as would be understood by a person of skill in the art.

As used herein, the term "expression system" relates to an *in vivo* or *in vitro* biological system that may be used to produce an expression product from a nucleic acid.

CRISPR techniques can also increase gene expression. CRISPR activation (CRISPRa) facilitates this for native genes by fusing relevant transcriptional activators to an inactive Cas9 that targets promoter or enhancer regions. Additionally, CRISPR Cas9-mediated gene editing leverages the homology directed repair (HDR) DNA repair mechanism to insert a desired gene from a repair template into a chosen site in a nucleic acid molecule which has been cut with the Cas9, directed to a specific sequence by the guide RNA. Strong promoters may also be inserted upstream of endogenous genes to enhance their expression, and chemical-inducible systems using promoters which are sensitive to an active agent may also be used. Such techniques will be familiar the skilled person and readily adapted to work certain embodiments of the invention.

As used herein, the term "overexpress" in the context of native genes relates to the increased expression of a native gene within a cell, organism, or other expression system so as to produce an elevated level of its genetic product. The genetic product may be the transcribed RNA, or the protein translated from the transcribed RNA. The skilled person will be familiar with methods to achieve such overexpression.

As used herein, the term "native" in the context of genes relates to a gene originating from a cell or organism of concern. Native genes can be understood to be endogenous genes.

As used herein, the term "density" relates to the mass of a particular substance per unit of volume. In the context of the extracellular vesicles of the invention, this translates to having a greater proportion of whole mitochondria or mitochondrial biomolecules compared to the volume of the extracellular vesicle.

The extracellular vesicles of the invention comprise an increased density of whole mitochondria or mitochondrial biomolecules.

As used herein, the term "whole mitochondria" relates to the complete mitochondrial organelle. As used herein, the term "mitochondrial biomolecules" relate to any substance that is produced by mitochondria and are used within biological processes within the mitochondria. In an embodiment, the mitochondrial biomolecules comprise mitochondrial DNA, mitochondrial RNA, mitochondrial proteins, mitochondrial lipids or mitochondrial polysaccharides, or mitochondrial peptides.

As used herein, the term "equivalent cell" relates to a cell to which the properties of the genetically modified cells of the present invention can be compared. This cell may also be referred to as a control cell, or an unmodified cell. The equivalent cell may be a cell from the same cell line or lineage or source or sample as the genetically modified cell and thus, prior to the modification of the genetically modified cell, may be essentially identical to the cell to be modified. The equivalent cell may not have been engineered to express at least two exogenous genes encoding at least two proteins of interest or may not have been engineered to overexpress at least two native genes encoding at least two proteins of interest. Therefore, the equivalent cell may be a clone of the genetically modified cell of the invention prior to its genetic modification. The skilled person will understand suitable equivalent cells to use as a baseline to compare the cells of the invention with, and extracellular vesicles derived from such cells with. The increase in particular cell components, such as whole mitochondria or mitochondrial biomolecules, in the cells or the extracellular vesicles of the invention, may be described as "enriched" in comparison with equivalent cells and extracellular vesicles derived therefrom.

Particular proteins of interest identified by the inventors comprise PGC-1α and PGC-1β. Peroxisome proliferator-activated receptor (PPAR) coactivator 1 (PGC-1) family proteins are transcriptional coactivators that assist in coordinating many physiological adaptations in various tissues. Often these responses are required due to increased demands for higher nutrient and energy supply. PGC-1α is a central factor in maintaining mitochondrial and energy metabolism homeostasis, and its activation is caused by a variety of stimuli such as AMPK, cAMP and cGMP, p38, SIRT1 and calcineurin. Their activation results in transcriptional co-activation of various genes involved in increasing the capacity for cellular energy production, one response of which includes stimulating mitochondrial biogenesis.

In any applicable embodiment, the PGC-1α encoding gene may comprise the nucleic acid sequence of SEQ ID NO: 1. In any applicable embodiment, a PGC-1α isoform may be used, and therefore a nucleic acid sequence encoding the following PGC-1α isoform amino acid sequences may be used: NCBI RefSeq NP_001341754.1 (SEQ ID NO: 39, isoform 1), NCBI RefSeq NP_037393.1 (SEQ ID NO: 40, isoform 2), NCBI RefSeq NP_001317681.1 (SEQ ID NO: 41, isoform 3), NCBI RefSeq NP_001317682.1 (SEQ ID NO: 42, isoform 4), NCBI RefSeq NP_001341756.1 (SEQ ID NO: 43, isoform 5), or NCBI RefSeq NP_001341757.1 (SEQ ID NO: 44, isoform 6).

In any applicable embodiment, the PGC-1β encoding gene may comprise the nucleic acid sequence of SEQ ID NO: 2. In any applicable embodiment, a PGC-1 β isoform may be used, and therefore a nucleic acid sequence encoding the following PGC-1β isoform amino acid sequences may be used: NCBI RefSeq NP_573570.3 (SEQ ID NO: 45, isoform 1), NCBI RefSeq NP_001166169.1 (SEQ ID NO: 46, isoform 2), or NCBI RefSeq NP_001166170.1 (SEQ ID NO: 47, isoform 3).

In any applicable embodiment, the hTERT encoding gene may comprise the nucleic acid sequence of SEQ ID NO: 3. In any applicable embodiment, a hTERT isoform may be used, and therefore a nucleic acid sequence encoding the following hTERT isoform amino acid sequences may be used: NCBI RefSeq NP_937983.2 (SEQ ID NO: 48, isoform 1), or NCBI RefSeq NP_001180305.1 (SEQ ID NO: 49, isoform 2).

It will be understood by the skilled person that for any applicable nucleic acid sequence as disclosed herein, for any nucleic acid sequence encoding any of the amino acid sequences as disclosed herein, for any nucleic acid sequence encoding any of the proteins of interest as disclosed herein, or for any nucleic acid sequence encoding any of the aspect of the present invention as disclosed herein, stop codons may be added or removed depending on the intended effect or context of its expression. For example, stop codons may need to be excluded at the end of a coding sequence when creating a fusion protein construct. Alternatively, stop codons may be removed to ensure open reading frame continuity, and when expressing multiple genes from a single open reading frame. In this case, stop codons would be omitted, and ribosome skipping peptides may be introduced between the coding sequences.

It is envisaged that further proteins of interest may be selected to be expressed by exogenous genes encoding the further proteins of interest, or by overexpression of native genes encoding the proteins of interest in the cell. In an embodiment, the further proteins of interest are selected from the group comprising: PPAR, NRF2, NRF1, TFAM, DRP1, OPAC1, SIRT3, POLG, TOP1 MT, MRPL12, MRPS16, SIRT1, AMPK, FOXO3, KLOTHO, NeuroD1, BDNF, REST, and TERT. Preferably, the further protein of interest is selected from TERT, PPAR, NRF1 or NRF2. Preferably, the TERT protein is hTERT. Preferably, the PPAR protein is PPARγ. Thus, in a preferred embodiment the cell is engineered to express, or overexpress, the proteins: PGC-1α, PGC-1β and hTERT; PGC-1α, PGC-1β and PPARγ; PGC-1α, PGC-1β and NRF1; or PGC-1α, PGC-1β and NRF2.

In any applicable embodiment of the invention as described herein, the PPAR protein may be PPARα, PPARβ, PPARγ, or PPARδ.

In an embodiment, the extracellular versicles are selected from small extracellular vesicles or microvesicles.

Any increase in density of whole mitochondria or mitochondrial biomolecules that may be observed in the extracellular vesicles of the invention may be characterised by the measurement of said increase in comparison with extracellular vesicles produced by an equivalent cell. In an embodiment, the density of whole mitochondria or mitochondrial biomolecules in the isolated extracellular vesicle may have increased by at least 10%, at least 20%, at least 30%, at least 40%, at least 50%, at least 60%, at least 70%, at least 80%, at least 90%, at least 100%, at least 125%, at least 150%, at least 175%, or at least 200% compared to that of an extracellular vesicle produced from the equivalent cell. In an embodiment, the density of whole mitochondria or mitochondrial biomolecules in the isolated extracellular vesicle may have increased by at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, at least 2-fold, at least 2.5-fold, at least 3-fold, at least 4-fold, at least 5-fold, at least 8-fold, at least 10-fold, at least 15-fold, at least 20-fold, at least 25-fold, at least 50-fold, at least 100-fold, at least 200-fold, at least 300-fold, at least 400-fold, at least 500-fold, or at least 1000-fold compared to that of an extracellular vesicle produced from the equivalent cell.

Additionally, the density of expression products from the genes encoding the proteins of interest in the genetically modified cell may increase in the extracellular vesicles of the invention and may be similarly characterised by the measurement of said increase in comparison with extracellular vesicles produced by an equivalent cell. In an embodiment, the density of the expression products of the genes encoding the proteins of interest in the isolated extracellular vesicle may have increased by at least 10%, at least 20%, at least 30%, at least 40%, at least 50%, at least 60%, at least 70%, at least 80%, at least 90%, at least 100%, at least 125%, at least 150%, at least 175%, or at least 200% compared to that of an extracellular vesicle produced from the equivalent cell. In an embodiment, the density of the expression products of the genes encoding the proteins of interest in the isolated extracellular vesicle may have increased by at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, at least 2-fold, at least 2.5-fold at least 3-fold, at least 4-fold, at least 5-fold, at least 8-fold, at least 10-fold, at least 15-fold, at least 20-fold, at least 25-fold, at least 50-fold, at least 100-fold, at least 200-fold, at least 300-fold, at least 400-fold, at least 500-fold, or at least 1000-fold compared to that of an extracellular vesicle produced from the equivalent cell.

In an embodiment, the density of TERT (preferably hTERT), PPAR (preferably PPARγ), NRF1 or NRF2, may have increased by at least 10%, at least 20%, at least 30%, at least 40%, at least 50%, at least 60%, at least 70%, at least 80%, at least 90%, at least 100%, at least 125%, at least 150%, at least 175%, or at least 200% compared to that of an extracellular vesicle produced from the equivalent cell. In an embodiment, the density of TERT (preferably hTERT), PPAR (preferably PPARγ), NRF1 or NRF2 may have increased by at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, at least 2-fold, at least 2.5-fold, at least 3-fold, at least 4-fold, at least 5-fold, at least 8-fold, at least 10-fold, at least 15-fold, at least 20-fold, at least 25-fold, at least 50-fold, at least 100-fold, at least 200-fold, at least 300-fold, at least 400-fold, at least 500-fold, or at least 1000-fold compared to that of an extracellular vesicle produced from the equivalent cell. The skilled person will understand that this increase may also be observed in the genetically modified cell from which the extracellular vesicles are derived. In an embodiment, the genetically modified cell may comprise a like increased density of expression products from the genes encoding the proteins of interest compared to that of the equivalent cell.

By being engineered to express at least two exogenous genes encoding the proteins of interest or overexpress at least two native genes encoding the proteins of interest, the genetically modified cell may exhibit a measurable fold-change in expression level of the genes encoding the proteins of interest compared to an equivalent cell. In an embodiment, the genetically modified cell may comprise an increased expression level of the genes encoding the proteins of interest compared to that of the equivalent cell. In an embodiment, any protein of interest may show an increased level of expression by at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, at least 2-fold, at least 2.5-fold, at least 3-fold, at least 4-fold, at least 5-fold at least 7-fold at least 10-fold, at least 20-fold, at least 50-fold, at least 100-fold, at least 200-fold, at least 500-fold, or at least 1000-fold compared to that of an equivalent cell. Gene expression level can be measured using qPCR and analysed using the 2^{-ΔΔC}_{T} method, of which the skilled person will be familiar.

The skilled person will understand that in any one of these increases in particular components within the EVs described, increases in the same component may also be observed in the genetically modified cell from which the extracellular vesicles are derived. For example, in an embodiment, the genetically modified cell may comprise an increased density of any one of whole mitochondria, mitochondrial biomolecules, or expression products of the genes of interest, compared to that of the equivalent cell.

It is envisaged that the extracellular vesicles of the present invention may be derived from cells of multiple lineages, which have then been subjected to genetic modification. In an embodiment, the genetically modified cell is a mesenchymal stem cell (MSC), hematopoietic stem cell (HSC), adipose-derived stem cell (ADSC), perivascular stem cell (PSC) or synovial stem cell (SSC). Preferably, the genetically modified cell is a mesenchymal stem cell (MSC). In an embodiment, the genetically modified cell is selected from the group consisting of umbilical cord MSCs, umbilical cord blood MSCs, Wharton's jelly MSCs, placental MSCs, amniotic fluid MSCs, peripheral blood MSCs, bone marrow MSCs, adipocyte MSCs, dental pulp MSCs, synovial membrane MSCs, endometrium MSCs, skin MSCs, muscle MSCs, oral cavity MSCs, and periodontal ligament MSCs.

In a second aspect of the invention, there is provided a method of preparing an isolated extracellular vesicle, the method comprising:
(a) subjecting a cell to genetic modification such that the cell is engineered to express at least two exogenous genes encoding at least two proteins of interest or to overexpress at least two native genes encoding at least two proteins of interest, wherein the at least two proteins of interest comprise PGC-1α and PGC-1β;
(b) culturing the cell, such that the extracellular vesicle is released from the cell; and
(c) isolating the extracellular vesicle released from the cell.

It is envisaged that the extracellular vesicle may comprise an increased density of whole mitochondria or mitochondrial biomolecules compared to that of an extracellular vesicle produced from an equivalent cell, wherein the equivalent cell has not been engineered to express the at least two exogenous genes encoding the at least two proteins of interest, or wherein the equivalent cell has not been engineered to overexpress the at least two native genes encoding the at least two proteins of interest.

The cell to which this method may apply may be any cell type as described in the first aspect of the invention which has not been genetically modified.

The genes encoding the proteins of interest used in this method may comprise further genes encoding the proteins of interest in addition to encoding PGC-1α and PGC-1β, as described in the first aspect of the invention.

As used herein, the term "release" in the context of EVs relates to the process by which EVs leave the cells from which they are derived into the extracellular environment. EVs may be released through the mechanism of membrane budding, which may also be referred to as either "shedding" or "membrane blebbing", in which vesicles are formed through the deformation of the plasma membrane and bud off/detach to produce lipid bilayer-delimited particles.

It is envisaged that the method may comprise introducing genes encoding the proteins of interest via a nucleic acid which comprises said genes which encode the proteins of interest to create genetically modified cells. In an embodiment, the genetic modification comprises introducing into the cell one or more nucleic acid constructs comprising coding sequences for the proteins of interest. As used herein, the term "nucleic acid construct" relates to an engineered or synthetic nucleic acid sequence that is commonly used for research or therapeutic purposes which comprises specific sequences of interest to impart an intended function on the system to which it is introduced, such as protein expression or gene silencing.

The method may comprise introducing the nucleic acid into the cell by either transfection or transduction. As used herein, the term "transfection" relates to a technique used to introduce an exogenous nucleic acid into a cell which utilises physical or chemical methods and will be familiar to the skilled person. As used herein, the term "transduction" relates to a technique used to introduce an exogenous nucleic acid into a cell which utilises viral vectors and will be familiar to the skilled person.

As a result of introducing the nucleic acid comprising coding sequences for the proteins of interest, an increased level of expression may be observed in the genetically modified cell.

In an embodiment of the method, the increase in expression is transient. As used herein, the term "transient" in the context of gene expression relates to the duration and nature of expression, specifically that the introduced nucleic acid does not integrate into the host's genome and therefore is diminished during cell division and will be familiar to the skilled person. In an embodiment of the method, the increase in expression is stable. As used herein, the term "stable" in the context of gene expression relates to the duration and nature of expression, specifically that the introduced nucleic acid does integrate into the host's genome and therefore is maintained during cell division and will be familiar to the skilled person.

In an embodiment of the method, the nucleic acid is contained within a recombinant vector. As used herein, the term "recombinant vector" relates to any nucleic acid molecule engineered to carry and deliver genetic material into a target cell. The genetic material may be, for example, a gene which encodes a protein of interest.

Various specific methods of introducing nucleic acids into cells are envisaged, of which the skilled person would be familiar. In an embodiment of the method, the nucleic acid is introduced by electroporation, microinjection, sonoporation, gene gun, lipofection, calcium phosphate precipitation, or nanoparticle. In another embodiment of the method, the nucleic acid is introduced by baculovirus, lentivirus, retrovirus, adenovirus, adeno-associated virus, vaccinia virus, herpes simplex virus, or sendai virus.

Any nucleic acid construct of the fifth aspect of the invention may be used in the method of the second aspect of the invention.

For example, in an embodiment the nucleic acid construct comprises a nucleic acid sequence comprising coding sequences for at least two proteins of interest, wherein the at least two proteins of interest comprise PGC-1α and PGC-1β.

The nucleic acid construct may further comprise a coding sequence for one or more additional proteins of interest selected from the group comprising: PPAR, NRF2, NRF1, TFAM, DRP1, OPAC1, SIRT3, POLG, TOP1MT, MRPL12, MRPS16, SIRT1, AMPK, FOXO3, KLOTHO, NeuroD1, BDNF, REST, and TERT. Preferably, the additional protein of interest is selected from TERT, PPAR,NRF1 or NRF2. Preferably, the TERT protein is hTERT. Preferably, the PPAR protein is PPARγ. The nucleic acid construct may comprise a coding sequence for at least one, at least two, at least three, at least four, at least five, at least six, at least seven, at least eight, at least 9, or at least 10 of these proteins of interest in addition to PGC-1α and PGC-1β.

In an embodiment, the nucleic acid construct comprises coding sequences for PGC-1α, PGC-1β and TERT. In an embodiment, the nucleic acid construct comprises coding sequences for PGC-1α, PGC-1β and PPAR (preferably PPARγ). In an embodiment, the nucleic acid construct comprises coding sequences for PGC-1α, PGC-113 and NRF1. In an embodiment, the nucleic acid construct comprises coding sequences for PGC-1α, PGC-113 and NRF2. In an embodiment, the nucleic acid construct comprises coding sequences for PGC-1α, PGC-1β, PPAR (preferably PPARγ) and NRF1. In an embodiment, the nucleic acid construct comprises coding sequences for PGC-1α, PGC-1β, PPAR (preferably PPARγ) and NRF2.

As used herein, the terms "coding sequence" or "coding region" relate to a nucleic acid sequence that comprises a sequence of codons that encodes, and is thus transcribed and translated into, the indicated protein when the coding sequence is expressed in a cell or a cell-free expression system (i.e. read and translated into the protein through ribosomal translation machinery). As would be understood by a person of skill in the art, several alternative coding sequences may encode the same amino acid sequence. In an embodiment, the coding sequence is codon optimised for any choice expression system for any of the genes encoding the proteins of interest as described herein. As used herein, the term "codon optimisation" relates to the usage of optimal codons in a gene sequence to maximise the expression of the gene in a relevant expression system.

In an embodiment, the nucleic acid construct comprises a transposable element. As used herein, the term "transposable element" relates to a type of nucleic acid sequence which is mobile and can therefore change its position within the genome and will be familiar to the skilled person. A coding sequence for a protein of interest may be comprised within the transposable element.

The method of the second aspect of the invention comprises culturing the cell (i.e. the genetically engineered cell) such that the extracellular vesicle is released from the cell.

The cell may be cultured as part of a 2D culture or a 3D culture. In an embodiment, the 3D culture is maintained within a hollow fibre bioreactor-based three-dimensional culture system. Such culture systems have been shown to produce a 19-fold production increase of EVs over traditional 2D culture while retaining characteristic surface markers, morphology, size, and EV markers compared to 2D culture systems (Cao et al. 2020).

Various methods to optimise or stimulate the release of extracellular vesicles are envisaged. As used herein, the term "optimise" or stimulate" in relation to EV release relates to any method which enhances the release of EVs from a cell, such that an increased quantity or increased quality of EVs are released from a cell compared to a cell to which such a method is not applied.

Since cells as described herein undergo senescence after a certain number of passages, therefore limiting their expansion and production capability, it is desirable to enhance production without losing product quality to optimise EV output.

Additionally, placing the stem cells under stressors through temperature and oxygen manipulation enhances the production of anti-inflammatory and other desired chemokines and peptides (Haque et al. Scientific World Journal 2013) which may be useful in regenerative therapies.

In an embodiment of the method, the cell is cultured in a media comprising N-methyldopamine or norepinephrine. N-methyldopamine and norepinephrine have been shown to triple EV production without altering their therapeutic viability (Wang et al. 2020).

In an embodiment of the method, the cell is cultured under hypoxic, low glucose, starvation, or oxidative stress conditions.

The method may further comprise culturing the cells with an agent that may increase the activity of the proteins of interest as described herein. Such agents may, for example, stimulate upstream factors of the proteins of interest. AMPK, a metabolic sensor which plays a role in cellular energy homeostasis, is known to increase the transcriptional activity of PGC-1α and PGC-1β through their phosphorylation. The kinase/phosphorylation activity of AMPK is vastly increased through phosphorylation of its catalytic domain. Therefore, in an embodiment of the method, the cell is cultured with an agent which stimulates AMPK phosphorylation, preferably wherein the agent is selected from with resveratrol, salicylate, alpha-lipoic acid, adiponectin or leptin. In an embodiment of the method, the cell is cultured with an agent which stimulates the phosphorylation activity of AMPK, preferably wherein the agent is selected from with resveratrol, salicylate, alpha-lipoic acid, adiponectin or leptin.

Similarly, the metabolic sensor SIRT1 acts upstream of PGC-1α and PGC-1β and can deacetylate these coactivators to increase their transcriptional activity. Therefore, in an embodiment of the method, the cell is cultured with an agent which stimulates the deacetylation activity of SIRT-1, preferably wherein the agent is selected from with resveratrol, lactate, NAD+, fisetin, quercetin, and curcumin.

Multiple kinds of cell, to which any method as described herein may be subjected to, are envisaged. The cell may be a primary cell isolated directly from the tissues of any living organism or taken from a primary cell culture. Alternatively, the cell may be one taken from a cell line, such as any immortalised cell line or stem cell line. Commercial cell banks may also act as a source for standardised and characterised cell lines. The method may also be applied to a cell in an *in vivo, in vitro* or ex *vivo* context. For the case of *in vivo* contexts, a cell would not have to be isolated from a living organism, and instead can remain in its natural environment. For the case of ex *vivo* contexts, a cell would be isolated from a living organism and subjected to the methods of the invention outside of its natural environment. For the case of *in vivo* contexts, the methods of the invention would be performed on a cell outside of a living organism.

In a third aspect of the invention, there is provided a genetically modified cell, wherein the cell is engineered to express at least two exogenous genes encoding at least two proteins of interest or to overexpress at least two native genes encoding at least two proteins of interest, wherein the at least two proteins of interest comprise PGC-1α and PGC-1β.

The genetically modified cell of this aspect may be any cell type as described in the first aspect of the invention.

The genes encoding the proteins of interest used in the engineering of the genetically modified cell may comprise further genes which encode proteins in addition to PGC-1α and PGC-1β, as described in the first aspect of the invention, in particular TERT (preferably hTERT), PPAR (preferably PPARγ), NRF1 or NRF2.

The genetically modified cell of this aspect may comprise an increased density of whole mitochondria or mitochondrial biomolecules compared to that of an equivalent cell. Furthermore, said increase may be characterised by the measurement of said increase in comparison with an equivalent cell, as described in the first aspect of the invention.

In an embodiment, the genetically modified cell comprises an increased expression level of the genes encoding the proteins of interest compared to that of the equivalent cell. In an embodiment, any gene encoding a protein of interest may show an increased level of expression by at least 10%, at least 20%, at least 30%, at least 40%, at least 50%, at least 60%, at least 70%, at least 80%, at least 90%, at least 100%, at least 125%, at least 150%, at least 175%, or at least 200% compared to that of an equivalent cell. In an embodiment, any gene encoding a protein of interest may show an increased level of expression by 1.1-fold, at least 1.2-fold, at least 1.3-fold, at least 1.4-fold, at least 1.5-fold, at least 1.6-fold, at least 1.7-fold, at least 1.8-fold, at least 1.9-fold, at least 2-fold, at least 3-fold, at least 4-fold, at least 5-fold, at least 8-fold, at least 10-fold, at least 15-fold, at least 20-fold, at least 25-fold, at least 50-fold, at least 100-fold, at least 200-fold, at least 300-fold, at least 400-fold, at least 500-fold, or at least 1000-fold compared to that of an equivalent cell. The skilled person will understand that expression products produced from the expression of these genes encoding proteins of interest will also increase, and in an embodiment, these expression products show a like increase in density within the cell compared to an equivalent cell.

Extracellular vesicles may be produced and subsequently isolated from the genetically modified cells of this aspect of the invention.

In an embodiment, the extracellular vesicles obtained from the genetically modified cell comprise an increased density of whole mitochondria or mitochondrial biomolecules compared to that of an extracellular vesicle produced from the equivalent cell. Furthermore, said increase may be characterised by the measurement of said increase in comparison with extracellular vesicles produced by the equivalent cell, as described in the first aspect of the invention.

Any nucleic acid construct of the fifth aspect of the invention may be comprised within the cells of the third aspect of the invention. The skilled person will understand that such constructs may be used to produce the genetically modified cells of this aspect of the invention.

In a fourth aspect of the invention, there is provided a method of increasing the density of whole mitochondria or mitochondrial biomolecules in a cell, the method comprising subjecting the cell to genetic modification such that the cell is engineered to express at least two exogenous genes encoding at least two proteins of interest or to overexpress at least two native genes encoding at least two proteins of interest, wherein the at least two proteins of interest comprise PGC-1α and PGC-1β.

It is envisaged that the cell may comprise an increased density of whole mitochondria or mitochondrial biomolecules compared to that of an equivalent cell, wherein the equivalent cell has not been engineered to express the at least two exogenous genes encoding the at least two proteins of interest, or wherein the equivalent cell has not been engineered to overexpress the at least two native genes encoding at least two proteins of interest.

The method of the fourth aspect of the invention may be an *in vivo, in vitro,* or ex *vivo* method.

The cell to which this method may apply may be any cell type as described in the first aspect of the invention which has not been genetically modified.

The genes encoding proteins of interest used in this method may comprise further genes in addition to PGC-1α and PGC-1β, as described in the first aspect of the invention.

Any nucleic acid construct of the fifth aspect of the invention may be used in the method of the fourth aspect of the invention.

In a fifth aspect of the invention, there is provided a nucleic acid construct comprising a nucleic acid sequence comprising coding sequences for at least two genes which encode at least two proteins of interest, wherein the at least two proteins of interest comprise PGC-1α and PGC-1β.

The nucleic acid construct of the invention may comprise at least one of the nucleic acid sequences selected from SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 18, SEQ ID NO: 19, SEQ ID NO: 20, SEQ ID NO: 21, SEQ ID NO: 22, SEQ ID NO:23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, and/or SEQ ID NO: 38, or comprise a nucleic acid sequence which encodes the amino acid sequence of any one of SEQ ID NO: 14, SEQ ID NO: 15, SEQ ID NO: 16, SEQ ID NO: 17, SEQ ID NO: 18, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, and/or SEQ ID NO: 56.

The constructs provided by this aspect of the invention comprise genes encoding proteins of interest which may be introduced to a cell to increase its density of whole mitochondria or mitochondrial biomolecules compared to that of an equivalent cell that has not been transfected with the construct of the invention.

The nucleic acid construct may further comprise a coding sequence for one or more additional proteins of interest selected from the group comprising: PPAR, NRF2, NRF1, TFAM, DRP1, OPAC1, SIRT3, POLG, TOP1MT, MRPL12, MRPS16, SIRT1, AMPK, FOXO3, KLOTHO, NeuroD1, BDNF, REST, and TERT. Preferably, the additional protein of interest is selected from TERT, PPAR or NRF1. Preferably, the TERT protein is hTERT. Preferably, the PPAR protein is PPARγ. The nucleic acid construct may comprise a coding sequence for at least one, at least two, at least three, at least four, at least five, at least six, at least seven, at least eight, at least 9, or at least 10 of these proteins of interest in addition to PGC-1α and PGC-1β

In an embodiment, the nucleic acid construct comprises coding sequences for PGC-1α, PGC-1β and TERT. In an embodiment, the nucleic acid construct comprises coding sequences for PGC-1α, PGC-1β and PPAR (preferably PPARγ). In an embodiment, the nucleic acid construct comprises coding sequences for PGC-1α, PGC-1β and NRF1. In an embodiment, the nucleic acid construct comprises coding sequences for PGC-1α, PGC-113 and NRF2. In an embodiment, the nucleic acid construct comprises coding sequences for PGC-1α, PGC-1β, PPAR (preferably PPARγ) and NRF1. In an embodiment, the nucleic acid construct comprises coding sequences for PGC-1α, PGC-1β, PPAR (preferably PPARγ) and NRF2.

In an embodiment, the nucleic acid construct further comprises an open reading frame, which comprises at least one coding sequence for any one of the proteins of interest. Various configurations of the constituent coding sequences contained within the open reading frames comprised in the nucleic acid construct are envisaged. In an embodiment, the coding sequences for the proteins of interest are each comprised in separate open reading frames. In an alternative embodiment, the coding sequences for the proteins of interest are contained within a single open reading frame.

As used herein, the term "open reading frame" relates to a nucleic acid sequence comprising a continuous sequence of DNA or RNA codons that may be transcribed or translated, and that begin with a start codon and ends with a stop codon. Start codons may comprise ATG/AUG, and stop codons may comprise TAA/UAA, TAG/UAG or TGA/UGA. The open reding frame may comprise introns and exons (such as a portion of genomic DNA) or comprise only exons (such as cDNA or processed/mature RNA).

The nucleic acid construct may further comprise a promoter. As used herein, the term "promoter" relates to a sequence of DNA that an RNA polymerase can recognise, bind to, and initiate the RNA synthesis of any coding sequence downstream of the promoter. In an embodiment, the promoter is located upstream of the open reading frame. The promoter may be eukaryotic or prokaryotic. Both constitutive promoters and cell-specific promoters may be used in the nucleic acid construct of this aspect of the invention. Promoters of the same identity, or different identities, may be used when the nucleic acid construct comprises more than one open reading frame (i.e. two or more open reading frames may be under the control of the same or different promoters). In an embodiment, the promoter is selected from the group consisting of CMV promoter, SV40 promoter, EF-1α promoter, CAG promoter, UbC promoter, TK promoter, PGK promoter, GRP78 promoter, β-actin promoter. Preferably, the promoter is a CMV promoter. In certain embodiments, the promoter may comprise the nucleic acid sequence of any of SEQ ID NO: 6, SEQ ID NO: 7, SEQ ID NO: 8, SEQ ID NO: 9, or SEQ ID NO: 10.

The nucleic acid construct may further comprise a polyA signal. As used herein, the term "polyA signal" relates to a nucleic acid sequence that directs the cleavage of a synthesised mRNA molecule and the following addition of a poly(A) tail. In certain embodiments, the polyA signal may be a SV40 late signal, an rRG polyA signal, or a BGH polyA signal. In certain embodiments, the polyA signal may comprise a nucleic acid sequence selected from SEQ ID NO: 11, SEQ ID NO: 12 or SEQ ID NO: 22.

The nucleic acid construct may also comprise a ribosome skipping peptide. As used herein, the term "ribosome skipping peptide" relates to a class of peptide which can induce ribosome skipping between two coding sequences so as to result in the translation of two unbound proteins. Such peptides may be used to express two separate proteins from a single open reading frame. Ribosome skipping peptides may also be known as self-cleaving peptides. In an embodiment, the ribosome skipping peptides are 2A peptides, preferably T2A, P2A, E2A or F2A. In an embodiment, the ribosome skipping peptide is encoded by a nucleic acid sequence which encodes an amino acid sequence selected from EGRGSLLTCGDVEENPGP (SEQ ID NO: 14), ATNFSLLKQAGDVEENPGP (SEQ ID NO: 15), QCTNYALLKLAGDVESNPGP (SEQ ID NO: 16), or VKQTLNFDLLKLAGDVESNPGP (SEQ ID NO: 17). Optionally, the ribosome skipping peptide further comprises the linker comprising the amino acid sequence GSG. The ribosome skipping peptide may be encoded by the nucleic acid sequence which comprises any of SEQ ID NO: 18, SEQ ID NO: 19, SEQ ID NO: 20, or SEQ ID NO: 21.

In an embodiment, the ribosome skipping peptides are positioned between any two consecutive coding sequences for the proteins of interest when comprised in a single open reading frame.

The nucleic acid construct may further comprise a transposable element.

The nucleic acid construct may comprise a 5' terminal transposon inverted repeat sequence and a 3' terminal transposon inverted repeat sequence. In a particular embodiment (a) the 5' terminal inverted repeat sequence is a PiggyBac 5' terminal transposon inverted repeat sequence, preferably wherein the sequence is SEQ ID NO: 4; and (b) the 3' terminal transposon inverted repeat sequence is a PiggyBac 3' terminal transposon inverted repeat sequence, preferably wherein the sequence is SEQ ID NO: 5. As used herein, the term "inverted repeat sequence" relates to a sequence of nucleotides followed downstream, either immediately or after an intervening nucleotide(s) by its reverse complement. In the context of transposons, inverted repeat sequences define the boundaries of transposons, and are the site of transposase binding which facilitates synapsis of the transposon ends, leading to integration into target DNA.

The nucleic acid construct may comprise a transposase coding sequence, and optionally a promoter controlling the expression of said transposase. In an embodiment, the transposase is a PiggyBac transposase, preferably wherein the PiggyBac transposase is encoded by the nucleic acid sequence SEQ ID NO: 29 or SEQ ID NO: 30, or is encoded by a nucleic acid sequence which encodes for the amino acid sequences SEQ ID NO: 55 or SEQ ID NO: 56. As used herein, the term "transposase" relates to a class of enzymes which can bind to the end of a transposon and catalyse its transposition to another region of the genome.

The coding sequences of the proteins of interest may be arranged in the following orders when comprised in a single open reading frame: PGC-1α: PGC-1β, PGC-1β:PGC-1α.

Preferably, the nucleic acid construct further comprises coding sequences for PPAR (preferably PPARγ), NRF1 or NRF2. In these embodiments, the coding sequences of the proteins of interest may be arranged in the following orders when comprised in a single open reading frame: PGC-1α:PGC-1β:PPARγ, PGC-1β:PGC-1α:PPARγ, PGC-1α:PPARγ:PGC-1β, PGC-1β:PPARγ:PGC-1α, PPARγ:PGC-1α:PGC-1β, PPARγ:PGC-1β:PGC-1α, PGC-1α:PGC-1β:NRF1, PGC-1β:PGC-1α:NRF1, PGC-1α:NRF1:PGC-1β, PGC-1β:NRF1:PGC-1α, NRF1:PGC-1α:PGC-1β, NRF1:PGC-1β:PGC-1α, PGC-1α:PGC-1β:PPARγ:NRF1, PGC-1α:PGC-1β:NRF1:PPARγ, PGC-1α:PGC-1β:NRF2, PGC-1β:PGC-1α:NRF2, PGC-1α:NRF2:PGC-1β, PGC-1β:NRF2:PGC-1α, NRF2:PGC-1α:PGC-1β, NRF2:PGC-1β:PGC-1α, PGC-1α:PGC-1β:PPARγ:NRF2, PGC-1α:PGC-1β:NRF2:PPARγ. The skilled person will understand that the coding sequences encoding the proteins of interest in these arrangements may be interspaced with additional non-coding sequences, such as the ribosome skipping peptides or linkers as described herein.

The nucleic acid construct may comprise a spacer region. As used herein, the term "spacer region" relates to a sequence of nucleic acids which separates structural or functional domains within a nucleic acid sequence.

In embodiments where the nucleic acid construct comprises the coding sequence for PGC-1α, the coding sequence may comprise the nucleic acid sequence SEQ ID NO: 1. Alternatively, the coding sequence may comprise a nucleic acid sequence which encodes for a PGC1α isoform amino acid sequence selected from SEQ ID NO: 39, SEQ ID NO: 40, SEQ ID NO: 41, SEQ ID NO: 42, SEQ ID NO: 43, or SEQ ID NO: 44,

In embodiments where the nucleic acid construct comprises the coding sequence for PGC-1β, the coding sequence may comprise the nucleic acid sequence SEQ ID NO: 2. Alternatively, the coding sequence may comprise a nucleic acid sequence which encodes for a PGC1β isoform amino acid sequence selected from SEQ ID NO: 45, SEQ ID NO: 46, or SEQ ID NO: 47.

In embodiments where the nucleic acid construct comprises the coding sequence for hTERT, the coding sequence may comprise the nucleic acid sequence SEQ ID NO: 3. Alternatively, the coding sequence may comprise a nucleic acid sequence which encodes for a hTERT isoform amino acid sequence selected from SEQ ID NO: 48 or SEQ ID NO: 49.

The nucleic acid construct may comprise the sequence of any of SEQ ID NO: 31, SEQ ID NO: 32, SEQ ID NO: 33, SEQ ID NO: 34, or SEQ ID NO: 35.

Also provided are nucleic acid constructs comprising the sequence of any of SEQ ID NO: 36, SEQ ID NO: 37, or SEQ ID NO: 38.

In an embodiment, the nucleic acid construct may comprise a selectable marker. As used herein, the term "selectable marker" relates to a nucleic acid that allows the identification or selection of a molecule or a cell that contains it against one that does not. The selectable marker may allow the production of a protein, peptide, RNA, or alternatively provide a binding site for proteins, peptides, RNA, or inorganic and organic compounds. The selectable marker may be one that encodes for antibiotic resistance, such as puromycin or ampicillin. The selectable marker may be one that encodes for a fluorescent protein, such as EGFP or mCherry. The selectable marker may be one that encodes for an enzyme which allows for luminescent identification, such as luciferase. In an embodiment, the selectable marker is encoded by any of SEQ ID NO: 23, SEQ ID NO: 24, SEQ ID NO: 26, SEQ ID NO: 27 or SEQ ID NO: 28. Alternatively, the selectable marker is encoded by a nucleic acid sequence which encodes for the amino acid sequence selected from SEQ ID NO: 50, SEQ ID NO: 51, SEQ ID NO: 52, SEQ ID NO: 53, or SEQ ID NO: 54.

The nucleic acid according to the fifth aspect of the invention may be comprised within a recombinant vector.

The nucleic acid construct of the fifth aspect of the invention may be comprised within many different types of recombinant vectors. In an embodiment, the recombinant vector is a plasmid, cosmid, fosmid, bacteriophage vector, bacterial artificial chromosome, yeast artificial chromosome, or Ti plasmid.

The recombinant vector may be a viral vector, a recombinant eukaryotic expression plasmid, or a recombinant cloning vector.

In embodiments where the recombinant vector is a recombinant viral vector, the recombinant viral vector may be a recombinant baculoviral vector, a recombinant lentiviral vector, a recombinant retroviral vector, a recombinant adenoviral vector, a recombinant adeno-associated viral vector, a recombinant vaccinia viral vector, a recombinant herpes simplex viral vector, or a recombinant sendai viral vector.

In a sixth aspect of the invention, there is provided a host cell comprising the nucleic acid according to the fifth aspect of the invention.

It is envisaged that the host cell could be selected from a wide variety of organisms. In an embodiment, the host cell is eukaryotic. In a further embodiment, the host cell is a mammalian cell, insect cell, or fungus. In an embodiment, the host cell is a bacterium. In a further embodiment, the host cell is selected from the bacterial genus *Escherichia, Pseudomonas, Streptomyces, Corynebacterium,* or *Brevibacterium.*

In a seventh aspect of the invention, there is provided a cell-free expression system comprising the nucleic acid according to the fifth aspect of the invention.

In an eighth aspect of the invention, there is provided a pharmaceutical composition comprising the isolated extracellular vesicle according to the first aspect of the invention, the genetically modified cell according to the third aspect of the invention, the nucleic acid construct according to the fifth aspect of the invention, the host cell according to the sixth aspect of the invention, or the cell-free expression system according to the seventh aspect of the invention.

It is envisaged that the pharmaceutical composition of this aspect of the invention may be in either an aqueous form or a lyophilised form.

It is envisaged that the composition may further comprise a pharmaceutical acceptable carrier or excipient.

As used herein, the term "pharmaceutically acceptable" relates to any substance intended for use in contact with tissues of animals that does not pose any reasonable risk of unwanted side effects, such as, but not limited to: toxicity, irritation and allergic response.

As used herein, the term "carrier" relates to substrates used in the process of drug delivery that improve the selectivity, effectiveness, and/or safety of drug administration. In an embodiment, the pharmaceutically acceptable carrier is a nanoparticle, microsphere, nanosphere, or nanocapsule. In an embodiment, the pharmaceutically acceptable carrier is a lipid-based nanoparticle, a polymer-based nanoparticle, an inorganic-based nanoparticle, peptide-based particle, a protein-based particle, a virus-like particle, or an emulsion. In an embodiment, the pharmaceutically acceptable carrier is a liposome, lipoplex, exosome, micelle.

As used herein, the term "excipient" relates to a pharmacological and immunologically inert substance that can be added to an active substance to enhance the stability or delivery of such active substance. In an embodiment, the pharmaceutically acceptable excipient is a solvent, buffer, binder, adjuvant, surfactant, lubricant, emulsifier, saline solution, preservative, pH adjuster, or any combination thereof.

The pharmaceutical composition may be formulated in varying ways to allow for different administration routes. In an embodiment, the pharmaceutical composition is formulated for intramuscular, intravenous, cutaneous, subcutaneous, intradermal, intrathecal, intraocular, intranasal, oral, intraaural, buccal, sublingual, or transdermal delivery, preferably intranasal delivery.

In an embodiment, the pharmaceutical composition is formulated for administration as a liquid, powder, capsule, tablet, or chewable tablet. In an embodiment, the composition is formulated for inhalation or nebulisation.

In addition, the pharmaceutical composition may be formulated so as to allow either the rapid, continuous or delayed release after administration, using any suitable method of preparation to facilitate such.

In a preferred embodiment, the pharmaceutical composition is formulated for intranasal delivery. The pharmaceutical composition may further comprise lyophilised isolated extracellular vesicles according to the first aspect of the invention. Such pharmaceutical compositions may be termed intranasal lyophilised vesicles, or ILVs. ILVs can offer numerous benefits for the brain, including regeneration of neural cells, repair of damage, reduction of inflammation, reversal of cell senescence, reduced amyloid plaque formation, enhanced blood vessel formation, reduction in fibrosis, prevention of cell death from trauma, and homing specificity to areas of damage. ILVs can reverse the ageing process, as indicated by a recent study by Greco et al. 2021 who restored aged blood by culturing with young umbilical cord extracellular vesicles, showing that miRNA cargo of microvesicles decreased inflammation, restored NK and other immune cells, and reversed cellular senescence.

ILVs represent a safe biologic which may be used to treat a variety of conditions, with various MSC EV treatments having been developed for the skin, joints, muscle, pancreas, and brain are being translated from preclinical models into early phase human clinical trials with proven safety. (Yin et al. Biomarker Research 2019). Nebulised MSC-EVs were well tolerated, and no serious adverse events were observed when used for lung injury (Shi et al. Journal of Extracellular Vesicles 2021). Injectable EVs are safer than stem cell administration as there is no risk of pulmonary embolism or cell differentiation, but ILV are used intra-nasally so carry no risk of infection by injecting through the dermal layer. Furthermore, ILVs, due to their dried state, can be produced in large quantities and stored until ready to use with minimal risk of contamination and structural damage during transit and delivery compared to fresh frozen.

Intranasal formulations of the pharmaceutical composition of the invention may comprise either liquid or powder formulations.

In an embodiment, the pharmaceutical composition is formulated as a spray, preferably for intranasal delivery.

Specific pharmaceutical compositions may be formulated based on a variety of factors, including but not limited to gender, age, body weight, sensitivity to drugs, disease severity, treatment period and treatment frequency.

In a ninth aspect of the invention, there is provided a kit comprising isolated extracellular vesicle according to the first aspect of the invention, the genetically modified cell according to the third aspect of the invention, the nucleic acid construct according to the fifth aspect of the invention, the host cell according to the sixth aspect of the invention, the cell-free expression system according to the seventh aspect of the invention, or the pharmaceutical composition according to the eighth aspect of the invention.

Certain embodiments of the invention relate to kits that comprise one or more components required to practice the invention.

The kit may further comprise instructions for use, particularly those regarding use in the treatment of an age-related or neurodegenerative disease. In an embodiment, the neurodegenerative disease may be selected from the group consisting of Alzheimer's disease, dementia, multiple sclerosis, amyotrophic lateral sclerosis, Parkinson's disease, Huntington's disease, multiple system atrophy, spinal muscular atrophy, cerebral palsy, Rett syndrome, and epilepsy. In an embodiment, the age-related disease may be selected from the group consisting of cancer, cardiovascular diseases, metabolic disorders, joint disorders including osteoarthritis, ocular disorders, immune system disorders, digestive and gastrointestinal disorders and dermal disorders.

In embodiments where the kit contains the nucleic acid construct according to the fifth aspect of the invention, the kit may further comprise a transfection medium, a transfection reagent, or a complexation medium. The kit may also comprise cells for transfection.

In an embodiment, one or more of the kit components are provided as multiple aliquots. Aliquots may be provided in a solid form, such as a frozen or lyophilized pellet. In an embodiment, the aliquot comprises the nucleic acid construct according to the fifth aspect of the invention as a transfection complex.

It is envisaged the products provided by the invention as disclosed herein will be of therapeutic utility. As used herein, the term "therapy" relates to the treatment of any disease, disorder, impairment or injury. As used herein, the term "treatment" relates to clinical treatments made to alter the course of disease in the absence of such treatment in an individual or cell. Treatment effects may comprise, reduction of disease progression, inhibition of the occurrence or recurrence of a disease, alleviation of symptoms of a disease, and alleviation of symptoms, or direct or indirect pathological consequences, of a disease.

In a tenth aspect of the invention, there is provided the isolated extracellular vesicle according to the first aspect of the invention, the genetically modified cell according to the third aspect of the invention, the nucleic acid construct according to the fifth aspect of the invention, the host cell according to the sixth aspect of the invention, the cell-free expression system according to the seventh aspect of the invention, the pharmaceutical composition according to the eighth aspect of the invention, or the kit according to the ninth aspect of the invention, for use in medicine. The products of the invention as described herein are also provided for prophylactic or therapeutic use in a subject. Also contemplated are their use in diagnostics. The diagnostics may be *in vivo, in vitro,* or ex *vivo.*

As used herein, the term "prophylaxis" relates to a preventative healthcare that focuses on the preservation of health, and any reference to medical use includes both prophylactic and therapeutic use. As used herein, the term "diagnostics" relates to any process concerned with the identification or characterisation of any disease or medical condition through analysing symptoms and any distinguishing features and may use medical devices or techniques for analysis.

Also provided herein is a method of treating a subject in need thereof, the method comprising administering to the subject the pharmaceutical composition as defined herein. As used herein, the term "subject" relates to any individual being observed or treated in a medical context. The subject may be a mammal, preferably a human.

In embodiments, the method comprises administering the pharmaceutical composition of the invention to a subject suffering from, having, susceptible to, or at risk of an age-related or neurodegenerative disease or symptom thereof. As used herein, the term "age related disease" relates to any disease which is caused by the physiological changes and decline in function that occur with advancing age. Such diseases often occur due to the cumulative effects of cellular damage and reduced capacity of cellular repair mechanisms observed during aging. As used herein, the term "neurogenerative disease" relates to any disease which can be is characterised by the progressive degeneration or loss of structure and function of neurons in the brain or peripheral nervous system. Evaluating whether a subject fits any of these criteria, and therefore would be in need of treatment, may be made through either an objective or subjective determination by a diagnostic test or opinion of a subject or health care provider (e.g. through identification of a relevant biomarker, genetic test, or family history).

As such, provided herein is a method of treating a subject with an age-related or neurodegenerative disease, the method comprising administering to the subject the pharmaceutical composition as defined herein.

In any applicable embodiment of the invention as described herein, the neurodegenerative disease may be selected from the group consisting of Alzheimer's disease, dementia, multiple sclerosis, amyotrophic lateral sclerosis, Parkinson's disease, Huntington's disease, multiple system atrophy, spinal muscular atrophy, cerebral palsy, Rett syndrome, and epilepsy.

In any applicable embodiment of the invention as described herein, the age-related disease may be selected from the group consisting of cancer, cardiovascular diseases, metabolic disorders, joint disorders including osteoarthritis, ocular disorders, immune system disorders, digestive and gastrointestinal disorders and dermal disorders.

A variety of delivery routes may be used to administer the pharmaceutical composition in the method. In an embodiment, the method comprises intramuscular, intravenous, cutaneous, subcutaneous, intradermal, intrathecal, intraocular, intranasal, oral, intraaural, buccal, sublingual, or transdermal delivery of the pharmaceutical composition of the invention, preferably intranasal delivery.

As part of the method, the pharmaceutical composition may be formulated as a liquid, powder, capsule, tablet for administration.

In an embodiment, the method comprises the administration of the pharmaceutical composition of the invention by an inhalation or nasal device. Such devices can facilitate the deposition of aerosolised formulations in the sinus cavity or alveoli of a subject. Examples of suitable devices include, nebulizers, dry powder generators, and sprayers.

The method may comprise the administration of a single dose of the pharmaceutical composition of the invention. Alternatively, the method may comprise a dosage regimen comprising multiple doses. In an embodiment, the interval between the first dosage and second dosage is at least 2, at least 3, at least 4, at least 5, at least 6, at least 7, at least 8, at least 9, at least 10, at least 11, at least 12, at least 26, at least 52, or at least 104 weeks. In an embodiment, the interval between the second dosage and any subsequent dosages is at least 2, at least 3, at least 4, at least 5, at least 6, at least 7, at least 8, at least 9, at least 10, at least 11, at least 12, at least 26, at least 52, or at least 104 weeks.

When the pharmaceutical composition of the invention comprises the EVs as described herein, suitable doses of the pharmaceutical composition of the invention include 1×10⁸, 2×10⁸, 3×10⁸, 4×10⁸, 5×10⁸, 6×10⁸, 7×10⁸, 8×10⁸, 9×10⁸, 1×10⁹, 2×10⁹, 3×10⁹, 4×10⁹, 5×10⁹, 6×10⁹, 7×10⁹, 8×10⁹, 9×10⁹, 1×10¹⁰, 2×10¹⁰, 3×10¹⁰, 4×10¹⁰, 5×10¹⁰, 6×10¹⁰, 7×10¹⁰, 8×10¹⁰, 9×10¹⁰, 1×10¹¹, 2×10¹¹, 3×10¹¹, 4×10¹¹, 5×10¹¹, 6×10¹¹, 7×10¹¹, 8×10¹¹, 9×10¹¹, 1×10¹², 2×10¹², 3×10¹², 4×10¹², 5×10¹², 6×10¹², 7×10¹², 8×10¹², 9×10¹², or 1×10¹³ EV particles per dose. The dose may be less than 1×10⁸ EV particles. The dose may be more than 1×10¹³ EV particles. Preferably, the administered dose is the minimum amount required to obtain the maximum effect while avoiding side effects.

The dosage administered may vary between individuals depending upon known factors, such as gender, age, body weight, sensitivity to drugs, disease severity, treatment period and treatment frequency. Additional factors include the mode and route of administration, the nature and extent of symptoms, and the final therapeutic effect desired.

The method may comprise administration of the pharmaceutical composition of the invention in isolation. Alternatively, the method may comprise an additional administration of a therapy or therapeutic agent. In one embodiment, the administration of an additional therapy or therapeutic agent is made prior to the administration of the pharmaceutical composition of the invention. In an alternate embodiment, the administration of an additional therapy or therapeutic agent is after the administration of the pharmaceutical composition of the invention. In another embodiment, the administration of an additional therapy or therapeutic agent is concurrent with the administration of the pharmaceutical composition of the invention, either co-formulated or administered in separate dosage forms. The additional therapy or therapeutic agent may be effective in treating or managing the symptoms of an age-related or neurodegenerative disease. In an embodiment, the additional therapeutic agent may in isolation be effective for the prevention or treatment of any age-related or neurodegenerative disease as described herein. The pharmaceutical composition of the invention and an additional therapeutic agent may be administered sequentially or simultaneously.

In some embodiments, the methods reduce outcomes associated with age related or neurodegenerative diseases, including impaired mental functioning, loss of muscle control, loss of memory, hallucinations, decline in cognitive function, social withdrawal, emotional blunting, disorientation, poor sleep, low energy levels, difficulty talking, chronic pain and stiffness, organ failure, increased mortality risk, increased infection risk, chronic inflammation, sensory impairment, physical weakness, reduced lifespan, impaired healing, and dependency on caregivers.

The methods of treatment as disclosed herein may be evaluated in their effectiveness by measuring treatment progression. The method may comprise a step of determining whether one or more symptoms of a particular disease has been reduced or eliminated due to the method of treatment. Alternatively, the method may comprise a step of determining the level of a diagnostic marker in a subject with an age-related or neurodegenerative disease. Any suitable diagnostic measurement, such as a screen or assay, may be used. When measuring the level of a diagnostic marker, the subject may or may not have been administered the pharmaceutical composition of the invention to treat an age-related or neurodegenerative disease. To monitor treatment progress, the presence of symptoms or a level of a diagnostic marker may be compared over time. In an embodiment, the method comprises determining the presence of a symptom or the level of a diagnostic marker in a subject with an age-related or neurodegenerative disease at a first and second timepoint. The first measurement may be made before the administration of the pharmaceutical composition of the invention, to obtain a pre-treatment level, which may be compared with the level obtained in the second measurement (i.e. after treatment). Alternatively, the first and second measurements may be taken from a subject which has already undergone treatment, to further monitor the progression of the treatment. The presence of a symptom or the levels of a diagnostic marker may be compared with each other, or to the level obtained from a (healthy) control subject, to determine the efficacy of the treatment.

The use of the isolated extracellular vesicle according to the first aspect of the invention, the genetically modified cell according to the third aspect of the invention, the nucleic acid construct according to the fifth aspect of the invention, the host cell according to the sixth aspect of the invention, the cell-free expression system according to the seventh aspect of the invention, the pharmaceutical composition according to the eighth aspect of the invention, or the kit according to the ninth aspect of the invention for the manufacture of a medicament for the treatment of an age-related or neurodegenerative disease is also included in the invention.

As used herein, the term "medicament" relates to any agent that may be used to assist in restoring health, and may treat, prevent, or alleviate the symptoms of a disease.

### Listed embodiments

The invention is further described with reference to the embodiments below: Provided herein is an isolated extracellular vesicle derived from a genetically modified cell, wherein the cell is engineered to express at least two exogenous genes encoding at least two proteins of interest or to overexpress at least two native genes encoding at least two proteins of interest, wherein the at least two proteins of interest comprise PGC-1α and PGC-1β.

In an embodiment, the extracellular vesicle comprises an increased density of whole mitochondria or mitochondrial biomolecules compared to that of an extracellular vesicle produced from an equivalent cell, wherein the equivalent cell has not been engineered to express the at least two exogenous genes encoding the at least two proteins of interest, or wherein the equivalent cell has not been engineered to overexpress the at least two native genes encoding the at least two proteins of interest.

In an embodiment further proteins of interest are selected from the group comprising: PPAR, NRF2, NRF1, TFAM, DRP1, OPAC1, SIRT3, POLG, TOP1MT, MRPL12, MRPS16, SIRT1, AMPK, FOXO3, KLOTHO, NeuroD1, BDNF, REST, and TERT. Preferably, the further protein of interest is selected from TERT, PPAR, NRF1 or NRF2. Preferably the TERT protein is hTERT. Preferably, the PPAR protein is PPARγ.

In an embodiment the extracellular versicles are selected from small extracellular vesicles or microvesicles.

In an embodiment the density of whole mitochondria or mitochondrial biomolecules in the isolated extracellular vesicle has increased by at least 10%, at least 20%, at least 30%, at least 40%, at least 50%, at least 60%, at least 70%, at least 80%, at least 90%, at least 100%, at least 125%, at least 150%, at least 175%, or at least 200% compared to that of an extracellular vesicle produced from the equivalent cell.

In an embodiment the density of TERT (preferably hTERT), PPAR (preferably PPARγ), NRF1 or NRF2 in the isolated extracellular vesicle has increased by at least 10%, at least 20%, at least 30%, at least 40%, at least 50%, at least 60%, at least 70%, at least 80%, at least 90%, at least 100%, at least 125%, at least 150%, at least 175%, or at least 200% compared to that of an extracellular vesicle produced from the equivalent cell.

In an embodiment the genetically modified cell is a mesenchymal stem cell (MSC) hematopoietic stem cell (HSC), adipose-derived stem cell (ADSC), perivascular stem cell (PSC) or synovial stem cell (SSC).

In an embodiment the genetically modified cell is selected from the group consisting of umbilical cord MSCs, umbilical cord blood MSCs, Wharton's jelly MSCs, placental MSCs, amniotic fluid MSCs, peripheral blood MSCs, bone marrow MSCs, adipocyte MSCs, dental pulp MSCs, synovial membrane MSCs, endometrium MSCs, skin MSCs, muscle MSCs, oral cavity MSCs, and periodontal ligament MSCs.

In an embodiment the genetically modified cell comprises an increased density of whole mitochondria or mitochondrial biomolecules compared to that of the equivalent cell.

In an embodiment wherein the mitochondrial biomolecules are mitochondrial DNA, mitochondrial RNA, mitochondrial proteins, mitochondrial lipids, mitochondrial polysaccharides, or mitochondrial peptides.

Also provided herein is a method of preparing an isolated extracellular vesicle, the method comprising:
(a) subjecting a cell to genetic modification such that the cell is engineered to express at least two exogenous genes encoding at least two proteins of interest or to overexpress at least two native genes encoding at least two proteins of interest, wherein the at least two proteins of interest comprise PGC-1α and PGC-1β;
(b) culturing the cell, such that the extracellular vesicle is released from the cell; and
(c) isolating the extracellular vesicle released from the cell.

In an embodiment, the extracellular vesicle comprises an increased density of whole mitochondria or mitochondrial biomolecules compared to that of an extracellular vesicle produced from an equivalent cell, wherein the equivalent cell has not been engineered to express the at least two exogenous genes encoding the at least two proteins of interest, or wherein the equivalent cell has not been engineered to overexpress the at least two native genes encoding the at least two proteins of interest.

In an embodiment the further proteins of interest are selected from the group comprising: PPAR, NRF2, NRF1, TFAM, DRP1, OPAC1, SIRT3, POLG, TOP1 MT, MRPL12, MRPS16, SIRT1, AMPK, FOXO3, KLOTHO, NeuroD1, BDNF, REST, and TERT. Preferably, the additional protein of interest is selected from TERT, PPAR, NRF1 or NRF2. Preferably the TERT protein is hTERT. Preferably, the PPAR protein is PPARγ.

In an embodiment the mitochondrial biomolecules are mitochondrial DNA, mitochondrial RNA, mitochondrial proteins, mitochondrial lipids, mitochondrial polysaccharides, or mitochondrial peptides.

In an embodiment the genetic modification comprises introducing into the cell one or more nucleic acid constructs comprising the coding sequences for the proteins of interest.

In an embodiment the nucleic acid is introduced via transfection or transduction.

In an embodiment the increase in expression of the genes encoding the proteins of interest is transient expression or stable expression.

In an embodiment the nucleic acid is contained within a recombinant vector.

In an embodiment the nucleic acid is introduced by electroporation, microinjection, sonoporation, gene gun, lipofection, calcium phosphate precipitation, or nanoparticle.

In an embodiment the nucleic acid is introduced by baculovirus, lentivirus, retrovirus, adenovirus, adeno-associated virus, vaccinia virus, herpes simplex virus, or sendai virus.

In an embodiment the nucleic acid comprises the nucleotide sequence of any of SEQ ID NO: 31, SEQ ID NO: 32, SEQ ID NO: 33, SEQ ID NO: 34, or SEQ ID NO: 35.

In an embodiment the cell is cultured in a media comprising N-methyldopamine or norepinephrine.

In an embodiment the cell is cultured under hypoxic, low glucose, starvation, or oxidative stress conditions.

In an embodiment the cell is cultured with an agent which stimulates AMPK phosphorylation, preferably wherein the agent is selected from with resveratrol, salicylate, alpha-lipoic acid, adiponectin or leptin.

In an embodiment the cell is cultured with an agent which stimulates the phosphorylation activity of AMPK, preferably wherein the agent is selected from with resveratrol, salicylate, alpha-lipoic acid, adiponectin or leptin.

In an embodiment the cell is cultured with an agent which stimulates the deacetylation activity of SIRT-1, preferably wherein the agent is selected from with resveratrol, lactate, NAD+, fisetin, quercetin, and curcumin.

Also provided herein is a genetically modified cell, wherein the cell is engineered to express at least two exogenous genes encoding at least two proteins of interest or to overexpress at least two native genes encoding at least two proteins of interest, wherein the at least two proteins of interest comprise PGC-1α and PGC-1β.

In an embodiment the further proteins of interest are selected from the group comprising: PPAR, NRF2, NRF1, TFAM, DRP1, OPAC1, SIRT3, POLG, TOP1MT, MRPL12, MRPS16, SIRT1, AMPK, FOXO3, KLOTHO, NeuroD1, BDNF, REST, and TERT. Preferably, the additional protein of interest is selected from TERT, PPAR, NRF1 or NRF2. Preferably the TERT protein is hTERT. Preferably, the PPAR protein is PPARγ.

In an embodiment the genetically modified cell is a mesenchymal stem cell (MSC), hematopoietic stem cell (HSC), adipose-derived stem cell (ADSC), perivascular stem cell (PSC) or synovial stem cell (SSC).

In an embodiment the genetically modified cell is selected from the group consisting of umbilical cord MSCs, umbilical cord blood MSCs, Wharton's jelly MSCs, placental MSCs, amniotic fluid MSCs, peripheral blood MSCs, bone marrow MSCs, adipocyte MSCs, dental pulp MSCs, synovial membrane MSCs, endometrium MSCs, skin MSCs, muscle MSCs, oral cavity MSCs, and periodontal ligament MSCs.

In an embodiment the genetically modified cell comprises an increased density of whole mitochondria or mitochondrial biomolecules compared to that of the equivalent cell.

In an embodiment the extracellular vesicles obtained from the genetically modified cell comprise an increased density of whole mitochondria, mitochondrial nucleic acids and/or mitochondrial nucleic acid products compared to that of an extracellular vesicle produced from the equivalent cell.

In an embodiment the mitochondrial biomolecules are mitochondrial DNA, mitochondrial RNA, mitochondrial proteins, mitochondrial lipids, mitochondrial polysaccharides, or mitochondrial peptides.

Also provided herein is an *in vitro* or ex *vivo* method of increasing the density of whole mitochondria or mitochondrial biomolecules in a cell, the method comprising subjecting the cell to genetic modification such that the cell is engineered to express at least two exogenous genes encoding at least two proteins of interest or to overexpress at least two native genes encoding at least two proteins of interest, wherein the at least two proteins of interest comprise PGC-1α and PGC-1β.

In an embodiment, the cell comprises an increased density of whole mitochondria or mitochondrial biomolecules compared to that of an equivalent cell, wherein the equivalent cell has not been engineered to express the at least two exogenous genes encoding the at least two proteins of interest, or wherein the equivalent cell has not been engineered to overexpress the at least two native genes encoding at least two proteins of interest.

Also provided herein is a nucleic acid construct comprising a nucleic acid sequence comprising coding sequences for at least two genes which encode at least two proteins of interest, wherein the at least two proteins of interest comprise PGC-1α and PGC-1β.

In an embodiment the nucleic acid construct further comprises the coding sequence for one or more additional proteins of interest selected from the group comprising: PPAR, NRF2, NRF1, TFAM, DRP1, OPAC1, SIRT3, POLG, TOP1MT, MRPL12, MRPS16, SIRT1, AMPK, FOXO3, KLOTHO, NeuroD1, BDNF, REST, and TERT. Preferably, the additional protein of interest is selected from TERT, PPAR, NRF1 or NRF2. Preferably the TERT protein is hTERT. Preferably, the PPAR protein is PPARγ.

In an embodiment the nucleic acid construct further comprises an open reading frame, which comprises at least one coding sequence for any one of the proteins of interest.

In an embodiment the coding sequences for the proteins of interest are each comprised in separate open reading frames.

In an embodiment the coding sequences for the proteins of interest are contained within a single open reading frame.

In an embodiment the nucleic acid construct further comprises comprising a promoter.

In an embodiment the promoter is located upstream of the open reading frame.

In an embodiment the promoter is a constitutive promoter or a cell-specific promoter.

In an embodiment the promoter is selected from the group consisting of CMV promoter, SV40 promoter, EF-1α promoter, CAG promoter, UbC promoter, TK promoter, PGK promoter, GRP78 promoter, or a β-actin promoter.

In an embodiment the promoter is a CMV promoter comprising the sequence SEQ ID NO: 7, SEQ ID NO: 8 or SEQ ID NO: 9.

In an embodiment the nucleic acid construct further comprises a ribosome skipping peptide, preferably wherein the ribosome skipping peptide is positioned between any two consecutive coding sequences for the proteins of interest when comprised in a single open reading frame.

In an embodiment the nucleic acid construct further comprises a 5' terminal transposon inverted repeat sequence and a 3' terminal transposon inverted repeat sequence.

In an embodiment:
(a) the 5' terminal inverted repeat sequence is a piggyBac 5' terminal transposon inverted repeat sequence, preferably wherein the sequence is SEQ ID NO: 4;
(b) the 3' terminal transposon inverted repeat sequence is a piggyBac 3' terminal transposon inverted repeat sequence, preferably wherein the sequence is SEQ ID NO: 5.

In an embodiment the nucleic acid construct further comprises a transposase coding sequence and a promoter controlling the expression of the transposase.

In an embodiment the transposase is a piggyBac transposase, preferably wherein the piggyBac transposase is encoded by the nucleic acid sequence SEQ ID NO: 29 or SEQ ID NO: 30, or is encoded by a nucleic acid sequence which encodes the amino acid sequences of SEQ ID NO: 55 or SEQ ID NO: 56.

In an embodiment, the coding sequences of the proteins of interest, when comprised in a single open reading frame, are arranged in the order of PGC-1α:PGC-1β, PGC-1β:PGC-1α, PGC-1α:PGC-1β:PPARγ, PGC-1β:PGC-1α:PPARγ, PGC-1α: PPARγ :PGC-1β, PGC-1β:PPARγ:PGC-1α, PPARγ: PGC-1α:PGC-1β, PPARγ:PGC-1β:PGC-1α, PGC-1α:PGC-1β:NRF1, PGC-1β:PGC-1α:NRF1, PGC-1α:NRF1:PGC-1β, PGC-1β:NRF1:PGC-1α, NRF1:PGC-1α:PGC-1β, NRF1:PGC-1β:PGC-1α, PGC-1α:PGC-1β:PPARγ:NRF1, or PGC-1α:PGC-1β:NRF1:PPARγ, PGC-1α:PGC-1β:NRF2, PGC-1β:PGC-1α:NRF2, PGC-1α:NRF2:PGC-1β, PGC-1β:NRF2:PGC-1α, NRF2:PGC-1α:PGC-1β, NRF2:PGC-1β:PGC-1α, PGC-1α:PGC-1β:PPARγ:NRF2, or PGC-1α:PGC-1β:NRF2:PPARγ..

In an embodiment, the nucleic acid construct comprises the nucleic acid sequence of any of SEQ ID NO: 31, SEQ ID NO: 32, SEQ ID NO: 33, SEQ ID NO: 34, or SEQ ID NO: 35.

In an embodiment the nucleic acid construct further comprises a selectable marker.

Also provided herein is a host cell comprising the nucleic acid construct as provided herein.

In an embodiment the host cell is the host cell is eukaryotic.

In an embodiment the host cell is a mammalian cell, insect cell, or fungus.

In an embodiment the host cell is a bacterium.

In an embodiment the host cell is selected from the bacterial genus *Escherichia, Pseudomonas, Streptomyces, Corynebacterium,* or *Brevibacterium.*

Also provided herein is a cell free expression system comprising the nucleic acid construct as provided herein.

Also provided herein is a pharmaceutical composition comprising the isolated extracellular vesicle as provided herein, the genetically modified cell as provided herein, the nucleic acid construct as provided herein, the host cell as provided herein, or the cell free expression system as provided herein, and a pharmaceutical acceptable carrier or excipient.

In an embodiment the pharmaceutical composition is in an aqueous form or a lyophilised form.

In an embodiment the pharmaceutically acceptable excipient is a solvent, buffer, binder, adjuvant, surfactant, lubricant, emulsifier, saline solution, preservative, pH adjuster, or any combination thereof.

In an embodiment the pharmaceutical composition is formulated for intramuscular, intravenous, subcutaneous, intradermal, intrathecal, intraocular, intranasal, oral, intraaural, or transdermal delivery, preferably intranasal delivery.

Also provided herein is a kit, comprising:
(a) the isolated extracellular vesicle as provided herein;
(b) the genetically modified cell as provided herein;
(c) the nucleic acid construct as provided herein;
(d) the host cell as provided herein;
(e) the cell free expression system as provided herein; or
(f) the pharmaceutical composition as provided herein;
and at least one of: instructions for use; a transfection medium; a transfection reagent; a complexation medium; or cells for transfection.

Also provided herein is the isolated extracellular vesicle as provided herein, the genetically modified cell as provided herein, the nucleic acid construct as provided herein, the host cell as provided herein, the cell free expression system as provided herein, or the pharmaceutical composition as provided herein, for use in medicine.

In an embodiment the isolated extracellular vesicle as provided herein, the genetically modified cell as provided herein, the nucleic acid construct as provided herein, the host cell as provided herein, the cell free expression system as provided herein, or the pharmaceutical composition as provided herein is for use in a method of treating an age-related or neurodegenerative disease.

In an embodiment the neurodegenerative disease is selected from the group consisting of Alzheimer's disease, dementia, multiple sclerosis, amyotrophic lateral sclerosis, Parkinson's disease, Huntington's disease, multiple system atrophy, spinal muscular atrophy, cerebral palsy, Rett syndrome, and epilepsy.

In an embodiment the age-related disease is selected from the group consisting of cancer, cardiovascular diseases, metabolic disorders, joint disorders including osteoarthritis, ocular disorders, immune system disorders, digestive and gastrointestinal disorders or dermal disorders.

In an embodiment the method comprises intramuscular, intravenous, subcutaneous, intradermal, intrathecal, intraocular, intranasal, oral, intraaural, or transdermal delivery.

### EXAMPLES

The invention is described with reference to the non-limiting Examples below:

### Example 1: Developing an experimental protocol to produce extracellular vesicles enriched in whole mitochondria or mitochondrial biomolecules

The present invention provides various products and methods which comprise EVs isolated from genetically modified cells, which may comprise an increased density of mitochondrial biomolecules compared to those isolated from a non-genetically modified cell, and production methods thereof. To accomplish this, a novel and inventive experimental protocol was developed to produce the isolated EVs, which could then be used on test cells, tissues, organisms and subjects with the aim of improving the function of neural tissues.

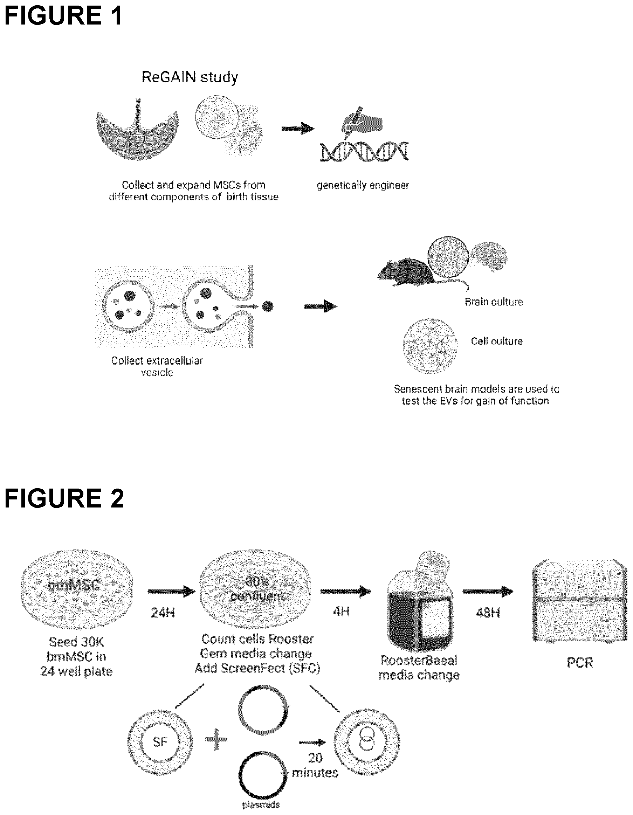

An example of this experimental protocol is described in Figure 1, which details how the cells from which the EVs will be derived and collected from may comprise MSCs from different components of birth tissue. Following this collection, the cells can be isolated and expanded until a suitable number has been reached. Following this, the cells are genetically modified using a selection of genes encoding proteins of interest to increase the density of whole mitochondria or mitochondrial biomolecules in the cell or EVs vesicles derived from the cell. Suitable genes encoding proteins of interest include those involved in mitochondrial biogenesis or metabolic regulation. Following this, EVs are collected from the genetically modified cells, and additional method steps may be introduced to increase the biogenesis of EVs to increase yield. Finally, the EVs may be suitably formulated and applied to test cells, tissues, organisms and subjects to help improve the function of neural tissues.

### Example 2: Developing an experimental protocol to introduce genes encoding proteins of interest into cells to stimulate the increased production of whole mitochondria or mitochondrial biomolecules

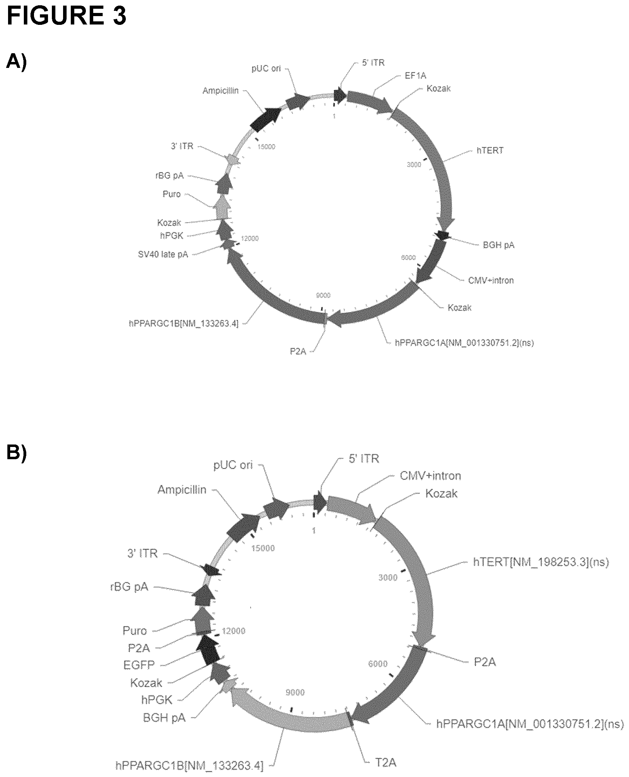

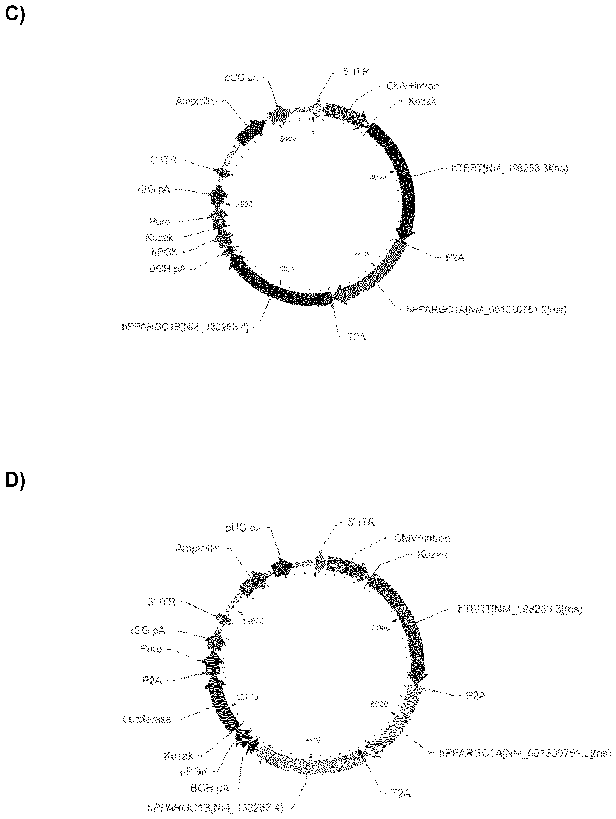

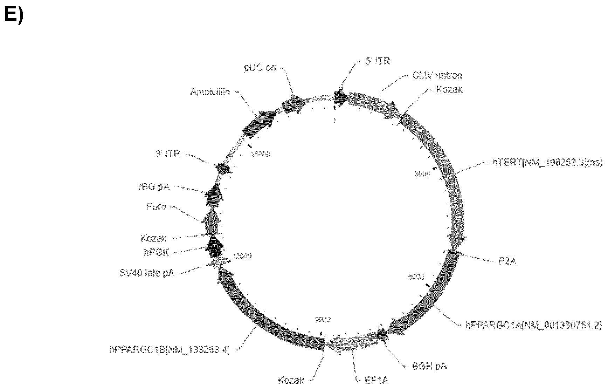

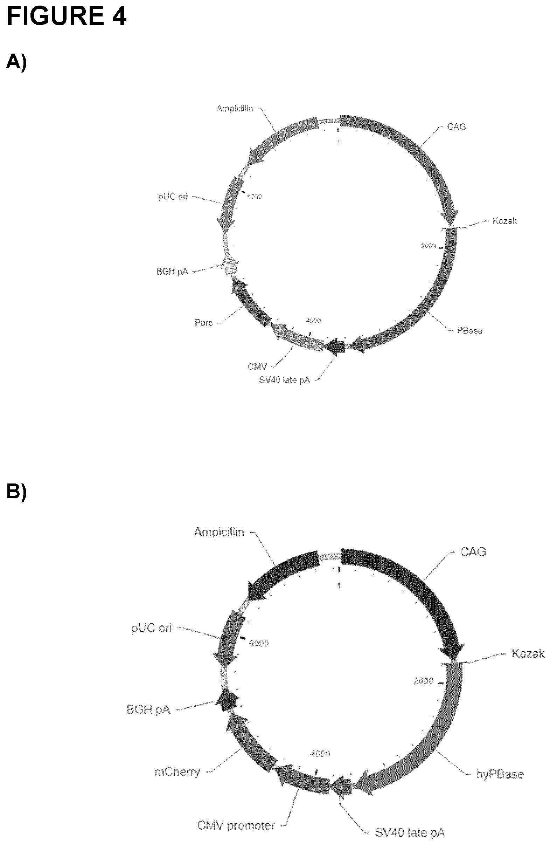

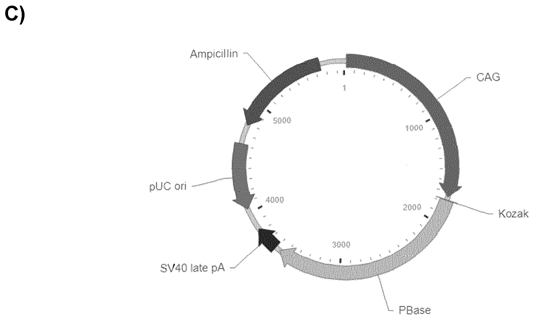

To introduce select genes into recipient cells, the PiggyBac^{™} transposon vector system was utilised to introduce three genes as a single transposable element. Plasmids were constructed comprising the three genes, hTERT, hPPARGC1A (PGC-1α) and hPPARGC1B (PGC-1β). Exemplary structures of constructs created which may be used to insert these genes are provided in Figure 3. In some constructs, all genes were under the control of a single promoter. In other constructs, more than one promoter was used for the expression of the genes. Additionally, separate plasmids were constructed comprising the PiggyBac^{™} transposase enzyme. Exemplary constructs comprising this transposase enzyme are provided in Figure 4.

A summary of the following protocol is provided in Figure 2.

Media was created in a Class II safety cabinet initially prepared by cleaning with 10% Chemgene^{™}, irradiation with UV light for 30 minutes, and finally cleaning with 70 % ethanol.

RoosterNourish^{™}-MSC components (RoosterBooster^{™}-MSC (Part No. SU-003) and RoosterBasal^{™}-MSC (Part No. SU-005/SU-022)) were brought to room temperature, protected from light, and left for up to four hours. Media was created by aseptically adding 1 bottle of RoosterBooster^{™}-MSC (Part No. SU-003) to 1 bottle of RoosterBasal^{™}-MSC (Part No. SU-005/SU-022) and combined well by capping and gently mixing the bottle. The final media preparation was aliquoted into 100 mL into sterile container

MSCs were then thawed and plated. 5 × 1 mL of MSC media was pre-warmed in a 15 mL conical tube. The cells were removed from liquid nitrogen and transferred to an ice container. Thawing was undertaken by holding the cryovial in 37°C water bath for approximately 1 minute. Once the cells had thawed, the cryovial was removed from the water bath, dried and sprayed with 70% ethanol. Cells were then transferred to a 15 mL centrifuge tube using a 1 mL pipette to remove cells from cryovial, and 6 mL of pre-warmed MSC medium was added slowly (drop by drop while gently swirling) into the cells. After centrifuging the cells at 300 × g for 5 min at room temperature, and medium was aspirated, leaving the cell pellet intact, which was subsequently resuspended in an appropriate volume of MSC medium based on the cell count. Cell counting was done using automated Countess II and mixing 10 µl of sample with 10 µl of trypan blue.

Transfection optimisation was then carried out for the plasmid constructs delivered by ScreenFect^{®} A in a 24-well plate. The plating density of htertMSC, ucMSC and bmMSC used in the optimisation assay are detailed in Table 1.

**Table 1: Plating density of htertMSC, ucMSC and bmMSC**

| **Culture wares** | **Surface area** | **Recommended MSC plating density at Thaw and seed cells at 10-15K cells/cm2 and allow for proliferation for up to ~4-24 hours or until >70% confluent.** |
|---|---|---|
| 24-well plate | 1.86 | 30,000 |
| 12-well plate | 3.5 | 50,000 |
| 6-well plate TF Nunclon | 9.6 | 125,000 |
| T-25 flask | 25 | Seed cells 5K/cm² |
| T-75 flask | 75 | Seed cells 5K/cm² |
| T-175 flask | 175 | Seed cells 5K/cm² |

MSCs were plated at 30K per well in a 24 well plate using RoosterNourish^{™} for 24h or until 80% confluence was reached. Media was then replaced ahead of use in the optimisation assay. Liposome complexes were suspended in RoosterCollect^{™} for 4 hours, then replaced with RoosterNourish^{™} media and incubated for 48 hours.

To prepare each well in the 24-well plate, initially 1(X) µL of ScreenFect^{®} A-plus was diluted in a dilution buffer to a final volume of 30 µl and mixed thoroughly, and 300(X) ng pDNA was diluted in a dilution buffer to a final volume of 30 µl. Then, the diluted ScreenFectA-plus and pDNA were combined and mixed immediately using 10 rapid pipette strokes. The SF-pDNA mixture was left for 20 minutes at room temperature for complex formation, without vortexing. 420 µl of freshly detached and resuspended cells were added to the 60 µl SF-pDNA complex and mixed with the pipette. Following this, the complex was added to a single well of 24 well plate. For adherent cells, media was removed and 420 µl of fresh media plus 60ul pDNA (SF-pDNA complex) were added. Plate layouts comprising specific solutions can be found in Tables 2a and 2b.

**Table 2a: Plating density of htertMSC, ucMSC and bmMSC**

| | | | | | |
|---|---|---|---|---|---|
| SFA-1µl pDNA-300nq | SFA-1.5µl pDNA-300ng | SFA-2µl pDNA-300ng | SFA-1µl pDNA-350ng | SFA-1.5µl pDNA-350ng | SFA-2ul pDNA-350ng |
| SFA-1µl pDNA-300nq | SFA-1.5µl pDNA-300ng | SFA-2µl pDNA-300ng | SFA-1µl pDNA-350ng | SFA-1.5µl pDNA-350ng | SFA-2ul pDNA-350ng |
| SFA-1µl pDNA-300ng | SFA-1.5µl pDNA-300ng | SFA-2µl pDNA-300ng | SFA-1µl pDNA-350ng | SFA-1.5µl pDNA-350ng | SFA-2ul pDNA-350ng |
| SFA-1µl pDNA-400nq | SFA-1µl pDNA-400ng | SFA-1µl pDNA-400ng | SFA-1.5µl pDNA-400ng | SFA-1.5µl pDNA-400ng | SFA-1.5ul pDNA-400ng |

**Table 2b: Plating density of htertMSC, ucMSC and bmMSC**

| | | | | | |
|---|---|---|---|---|---|
| SFA2µl pDNA-400nq | SFA-2µl pDNA-400ng | SFA-2µl pDNA-400ng | SFA-1.5µl pDNA-0ng | SFA-1.5µl pDNA-0ng | SFA-1.5 ul pDNA-0ng |
| SFA-0µl pDNA-350nq | SFA-0µl pDNA-350ng | SFA-0µl pDNA-350ng | SFA-0µl pDNA-0ng | SFA-0µl pDNA-0ng | SFA-0ul pDNA-0ng |
| Cell count -pre | Cell count-pre | Cell count-pre | Cell count -post | Cell count -post | Cell count -post |

### Example 3: Measurement of relative fold gene expression of introduced genes encoding proteins of interest to find optimal loading dose

Once cells had been transfected with the selected genes, their expression was measured relative to PPIA (Peptidylprolyl Isomerase A) using 2^{-ΔΔCt} analysis from qPCR measurements for a number of different liposome:plasmid ratios to identify the optimal loading conditions for the most desired expression profile.

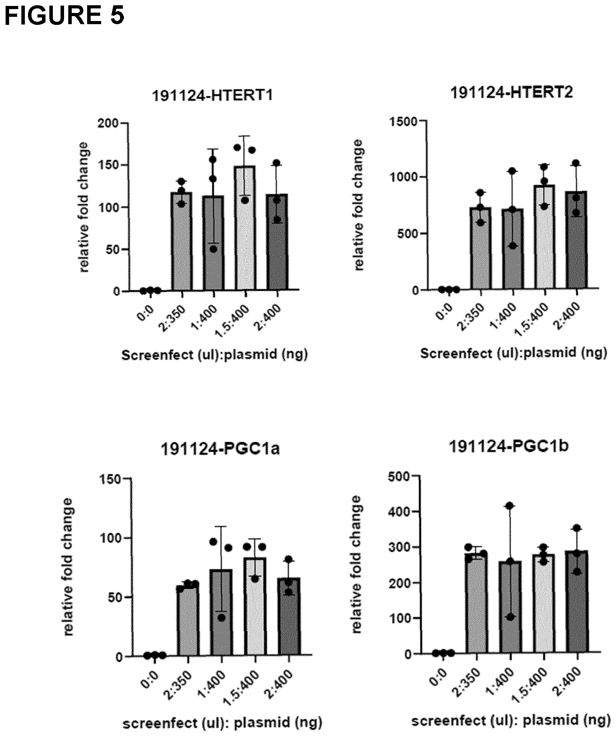

Figure 5 shows the relative fold gene expression of hTERT, PGC-1α and PGC-1β in ucMSCs transfected using the plasmid from Figure 3D and using PPIA as a housekeeping gene. From these results, the integration of the three genes produced balanced expression across each of hTERT, PGC-1α and PGC-1β for varying ratios of ScreenFect^{®} A (µl) to plasmid (ng). From these results, it has been demonstrated that up to three genes encoding proteins of interest (in this example, hTERT, PGC-1α and PGC-1β) can be introduced to a recipient cell via a single construct and show a strong increase in fold change in expression. The inventor proposes that the increase in expression of these genes may stimulate the mitochondrial biogenesis pathway and may increase the density of whole mitochondria or mitochondrial biomolecules in the genetically modified cells, and similarly may increase this density of whole mitochondria or mitochondrial biomolecules in the EVs produced by these cells.

### Example 4: Mesenchymal Stem Cell extracellular vesicle collection using IZON size exclusion chromatography

Following the MSC ScreenFect^{®}-A-Plasmid transfection protocol at appropriate dosage, EVs were harvested using the following method.

Media was prepared by allowing RoosterCollect^{™}-EV to warm to room temperature away from light for up to 4 hours. Following this, cell cultures were washed with prewarmed PBS to remove impurities and residuals from RoosterNourish^{™}. The spent medium was aspirated from cell culture flasks, and an equivalent working volume of RoosterCollect^{™}-EV was added. The flasks were then returned to the incubator (37°C, 5% CO₂) for up to 48 hours. After culture time, the conditioned media was harvested for particle collection.

IZON size exclusion chromatography was then carried out using Amicon^{®} Ultra Centrifugal filters 15 mL (Merck) and IZON columns (20 nm and 35 nm series).

Samples were centrifuged prior to loading onto the column. To avoid the clogging of column frits, it was recommended to filter or centrifuge the biological sample to remove large particulate matter. Centrifugation was undertaken at 1,500 × g for 10 minutes to remove any cells and large particles. The supernatant was gently transferred to a new tube and centrifuged again at 10,000 × g for 10 minutes.

Concentration was carried out via Amicon^{®} Ultra Centrifugal filters (Merck). Device equilibration was done by adding 2 mL PBS to the device, capping and centrifuging at 4,000 × g for 10 minutes in a swinging bucket rotor. The unfiltered PBS from the bottom of the filter device was removed, and the filtrate from the collection tube was aspirated.

For sample ultrafiltration, 15 mL of sample was added to the AU-15 filter and the device was capped. Centrifugation at 4,000 × g for 30 minutes was done and the device was then removed from the centrifuge and the collection tube emptied.

To recover the concentrated sample from the filter device, for AU-2, -4, and -15 devices, a pipettor (p200) was inserted to withdraw a volume of sample (200 µL), and the membrane was washed down the side walls multiple times (3-4 on each side) to ensure complete recovery. The extracellular vesicle sample is then ready for the IZON column. Note that concentration of samples using filtration after purification with qEV may result in the loss of some EVs, and that treating columns as single use is advisable where the vesicles will be analysed for nucleic acids. This will eliminate the possibility of cross-contamination between samples.

To maintain the functionality of EVs, the flushing buffer should be of the same temperature as the sample buffer. SEC can also be used to exchange the buffer of a sample. The sample buffer temperature should be within the operational temperature of 18-24 °C (65-75 °F), and further should be degassed and room temperature to avoid air bubbles forming in the resin bed. Rapid changes in temperature, for example removing packed columns from a cold room and applying buffer at room temperature, can introduce air bubbles in the packed bed, resulting in poorer separation. Use of a buffer with an ionic strength of 0.15 M or greater can help avoid any unwanted ionic interactions between the solute molecule and the matrix. Use of freshly filtered (0.22 µm) buffer can avoid introducing particulate contamination. qEV columns come equilibrated in filtered PBS containing < 0.1% ProClin 200 or < 0.1% w/v sodium azide.

For the column setup and equilibration, the sample buffer was made to be within the operational temperature range of 18-24 °C, and the column caps were not removed until the column has likewise reached the operational temperature. Upon removing the top cap, it was attached to the column in an upright position to a stand ready for use. Alternatively, Automatic Fraction Collectors (AFCs) and qEV racks are available commercially. The bottom cap was then removed and the buffer allowed to start running through the column. For column flushing, the buffer reservoir was attached to the top of the column, and the column flushed with at least two column volumes of PBS buffer to minimises potential effects of storage buffer on any downstream applications. If an elution buffer other than PBS is to be used, the column should be equilibrated with at least three column volumes of the new buffer. Only freshly filtered (0.22 µm) buffer should be used to avoid introducing particulate contamination.

For sample collection, IZON parameters were set to collect the number and volume of samples required (i.e. 5 samples at 400ul each). To avoid the clogging of column frits, it is recommended to filter or centrifuge the biological sample to remove large particulate matter. Buffer was continued to run through the column (the column will stop flowing when all of the buffer has entered the loading frit). 500 µl of the prepared centrifuged sample volume was loaded onto the loading frit. Stopping the column flow during the run for long periods of time should be avoided to ensure accurate EV separation. If sample is less than 500ul add filtered PBS to make up the volume to 500 µl. The buffer volume was collected (including the volume displaced by loading the sample). The sample was allowed to run through the column, and the column will stop flowing when all of the sample has entered the loading frit. Following this, the column was topped up with buffer and the collection of the buffer volume was continued. Once the buffer volume had been collected, the Purified Collection Volume (PCV) was then collected. To collect accurate volumes, only the required volume was loaded to the top of the column, and then waiting for that volume to run through until the flow stops before repeating. Final samples were stored at -80°C.

### Example 5: Characterisation and assessment of EVs

The characteristics of EVs of the invention may assessed through many different assays and methods depending on the desired factor to measure.

EVs may be characterised by their physical properties. The mitochondrial density of EVs may be measured using a MitoTracker assay, which uses a lipophilic cationic dye that is taken up by the mitochondria to allow fluorescent visualisation. Flow cytometry and imaging may be used for EV phenotyping, particle count, and morphology characterisation using fluorescent labelling. The size and distribution of EVs may be measured using zeta potential measurements and nanoparticle tracking analysis (NTA). Additionally dynamic light scattering (DLS) and electrophoretic light scattering (ELS) may be used to determine surface charge. This method may further measure size using Brownian motion and speed of particles in an electric field. Tunable resistive pulse sensing (TRPS) may be used to measure particle size and concentration based on electrical impedance.

The presence of certain constituent nucleic acids may be confirmed with PCR studies. Additionally, proteomics and lipidomics may further confirm the presence of any biomolecules of interest. Western blots may be used to identify particular marker proteins such as classical EV markers (CD63, CD9, CD81), markers of mitochondrial origin, or any products of the genes as disclosed herein.

Functional assays may also be used to probe the effects EVs may impart on recipient cells. The specific assay used will depend on the tested function, but examples of relevant tests include ROS assays to measure oxidative stress reduction, and cell viability/proliferation assays to measure the protective or growth-promoting effects of EVs.

The therapeutic effects of EVs may be studied using relevant animal models harbouring a relevant disease or condition, such as an age-related disease or neurodegenerative disease. Assessment of the impacts of EVs administered can be histological (to determine changes in tissue composition), molecular (to determine any changes in *in vivo* gene expression or signalling pathways after EV treatment), or functional (to evaluate systemic chances as a result of EV treatment, such as improved cognitive performance or memory).

### Example 6: Development of a senescent model to assess the effects of EVs

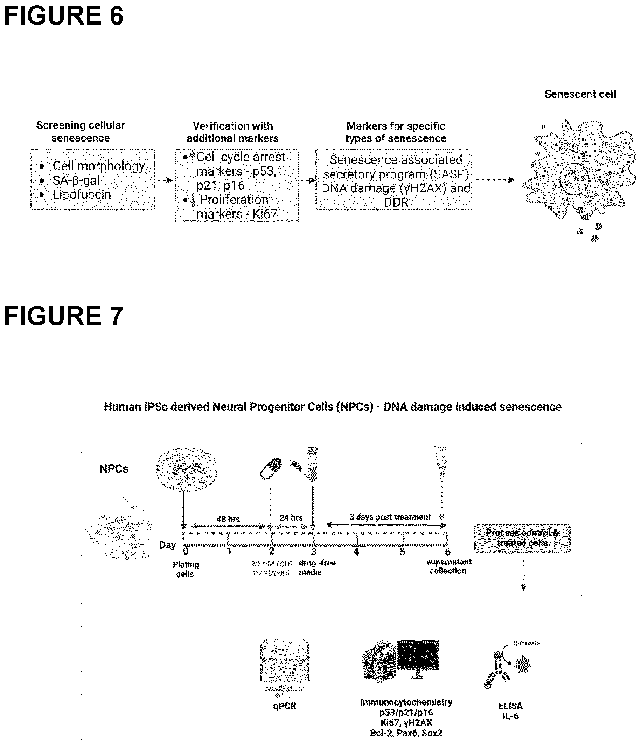

Measuring the potential gain of function effects of EVs of the invention may be tested on senescence models. By evaluating how EVs influence senescence markers, the efficacy of EVs of the invention in modulating senescence-related processes maybe assessed. An exemplary screening approach is provided in Figure 6.

### Example 7: protocols for preparation and culture of neural progenitor cells (NPC), human microglial cell line HMC3, and rat hippocampal cells

### NPCs

For preparation of 100 mL of STEMdiff^{™} Neural Progenitor Complete Medium, 98 mL of STEMdiff^{™} Neural Progenitor Basal Medium, 2 mL of STEMdiff^{™} Neural Progenitor Supplement A (50X), and 100 µL of STEMdiff^{™} Neural Progenitor Supplement B (1000X) are mixed.

Cultureware may be coated with Corning^{®} Matrigel^{®} with the following method. One aliquot of Corning^{®} Matrigel^{®} was thawed on ice. 24 mL of cold DMEM/F-12 is aliquoted with 15 mM HEPES into a 50 mL conical tube and kept on ice.

The thawed Matrigel is transferred onto the cold DMEM/F-12 with 15 mM HEPES and mixed well. The culture plate is coated with the diluted Matrigel solution and swirled to spread across the surface. The culture plate can be sealed with Parafilm and stored at 2 - 8°C for up to 1 week after coating. The culture plate is then incubated at room temperature for at least 1 hour before use.

The recommended volumes for coating culture plates with Corning^{®} Matrigel^{®} are as follows: 96-well plate: 50 µL/well, 24-well plate: 250 µL/well, 12-well plate: 500 µL/well, 6-well plate: 1 mL/well.

The NPCs may be plated as follows. Aliquot the required volume of DMEM/F-12 with 15 mM HEPES and STEMdiff^{™} Neural Progenitor complete medium and warm the tube(s) in a water bath set at 37°C. Remove the cells from liquid nitrogen and transfer to the workspace in a freezing container. Spray the vial with 70% ethanol and loosen the cap slightly in a clean Class II safety cabinet. Re-tighten the cap of the cryovial. Immediately hold cryovial in a 37°C water bath for approximately 2 minutes until the cells are thawed. Remove the cryovial from the water bath and wipe it with 70% ethanol. Transfer 20 µL of cell suspension to a 0.5 mL tube containing 20 µL of Trypan Blue for pre-wash counting. Mix by pipetting and load 10 µL into one side of a haemocytometer. Use a 1 mL pipette to remove cells from cryovial and transfer to a 15 mL centrifuge tube containing 10mL of pre-warmed DMEM/F-12 with 15 mM HEPES and mix gently. Centrifuge cells at 300 × g for 5 minutes at room temperature. Aspirate the medium, leaving the cell pellet intact. Resuspend the cell pellet in 2 mL STEMdiff^{™} Neural Progenitor complete medium. Transfer 20 µL of cell suspension to a 0.5 mL tube containing 20 µL of Trypan Blue for post-wash counting. Mix by pipetting and load 10 µL into one side of a haemocytometer. Calculate the volume of cells required to seed the wells. Gently remove the excess Matrigel solution from the culture plate. Transfer the required volume of cells into the wells. Spray the flask with 70% ethanol. Incubate cells at 37°C at 5% CO₂. Daily full-medium changes should be performed until the cells are ready to passage.

Passaging NPCs for expansion may be done as follows. Coat the desired number of wells with Matrigel solution (as in the above protocol). Aliquot the required volume of DMEM/F-12 with 15 mM HEPES and STEMdiff^{™} Neural Progenitor complete medium and warm the tube(s) in a water bath set at 37°C. Warm ACCUTASE^{™} to room temperature. Aspirate the spent medium and add the required volume of ACCUTASE^{™} to the wells (0.5 mL for 12 well culture plate). Incubate at 37°C and 5% CO2 for 10 minutes. Pipette the cell suspension up and down to dislodge remaining attached cells. Transfer the NPC suspension to a 15 mL conical tube containing 3 mL of DMEM/F-12 with 15 mM HEPES. Centrifuge the cells at 300 × g for 5 minutes at room temperature. Aspirate the supernatant and resuspend the cells in 1 mL STEMdiff^{™} Neural Progenitor complete medium. Transfer 20 µL of cell suspension to a 0.5 mL tube containing 20 µL of Trypan Blue for counting. Mix by pipetting and load 10 µL into one side of a haemocytometer. Calculate the volume of cells required to seed the wells. Gently remove the excess Matrigel solution from the culture plate. Transfer the required volume of cells into the wells and incubate at 37°C at 5% CO₂. The cultures may be visually assessed to monitor growth and to determine timing of the next passage (after approximately 7 days of culture).

NPCs may be cryopreserved as follows. Pre-warm the required volume of STEMdiff^{™} Neural Progenitor Freezing Medium. Perform NPC passaging protocol as detailed above to obtain cells. Transfer 1.5 - 6 × 10^10⁶ of viable cells per cryovial using 1 mL of STEMdiff^{™} Neural Progenitor Freezing Medium. Finally, transfer the cryovials into -80°C for 24h and then to liquid nitrogen.

### HMC3

The base medium of the HMC3 cell line ("HMC3 media") may be EMEM (Sigma, cat.no M0325). To make the complete growth medium, add 10% FBS, 1% penicillin-streptomycin (10,000 units penicillin and 10 mg streptomycin/mL), and 1mM sodium pyruvate solution (Sigma, S8636).

HMC3 cells may be taken from stock as follows. Aliquot the 9 mL of HMC3 complete medium into 15 mL centrifuge tubes and warm the tube(s) in a water bath set at 37°C for 30 min. Spray cryovial with 70 % ethanol and loosen the cap slightly in a clean Class II safety cabinet. Re-tighten the cap of the cryovial. Immediately hold the cryovial in 37°C water bath for approximately 2 minutes until the cells are thawed. Use a 1 mL pipette to remove the cells from cryovial and transfer to a 15 mL centrifuge tube containing 9 mL of pre-warmed HMC3 complete medium. Centrifuge the cells at 125 × g for 5 minutes at room temperature. Transfer 4 mL of pre-warmed HMC3 complete medium to a T25 flask. Discard the supernatant into a beaker containing chemgene. Resuspend the cells in 1 mL of pre-warmed HMC3 complete medium and transfer to the T25 flask containing 4 mL of pre-warmed HMC3 complete medium. Spray the flask with 70 % ethanol. Incubate the cells at 37°C at 5% CO₂ until the cells reach 70 - 80 % confluency.

HMC3 cells may be passaged as follows. The cells may be visualised daily using an inverted light microscope with a low power objective lens. The cells should be passaged when 70 - 80 % confluent and not be allowed to overgrow. Warm the PBS, trypsin-EDTA solution (0.25% trypsin - 0.53mM EDTA) and HMC3 complete medium in a water bath set to 37°C for 30 minutes. Spray gloves with 70 % ethanol and transfer the flask from the incubator to a clean Class II safety cabinet. Discard the spent medium from flask into a beaker containing chemgene. Wash the adherent cells in culture flask using 5 mL of PBS (T25 flask) or 10 mL of PBS (T75 flask) taking care not to pipette the PBS directly onto the cells. Wash the cells again using 1 mL of trypsin-EDTA solution (T25 flasks) or 3 mL of trypsin-EDTA solution (T75 flasks). Add 1 mL of trypsin-EDTA solution (T25 flasks) or 2 mL of trypsin-EDTA solution (T75 flasks) to cells and swirl the flask around to distribute the trypsin-EDTA solution evenly throughout the culture flask surface. Incubate the cells at 37°C at 5% CO₂ for 3 minutes. Cells will be seen lifting off after trypsin addition. Pat the short end of the flask to help detach any remaining attached cells. Visualise the cells using the inverted light microscope to ensure that they are detached. Add 4 mL of "HMC3 media" (T25 flask) or 8 mL of "HMC3 media" (T75 flask) to the flask and wash the base of the flask by pipetting. Collect the cells and transfer to a 15 mL centrifuge tube. Centrifuge at 125 × g for 5 minutes at room temperature. Discard the supernatant from the centrifuge tube and resuspend the cell pellet in each in 1 mL of "HMC3 media". Mix by pipetting up and down 3-5 times, ensuring that the cells do not become aerosolized. Transfer 20 µL of cell suspension to a 0.5 mL tube containing 20 µL of Trypan Blue (Sigma Aldrich, cat. no. T8154). Mix by pipetting and load 10 µL into a hematocytometer counting slide. Count the cells using a microscope and calculate the volume of cells required to seed flasks. For these cells, seeding densities should be 1 × 10⁶ for a T75 flask. Transfer the required volume of cells to a T25 flask or T75 flask. Use "HMC3 media" to make the total volume up to 5 mL (T25 flask) or 15 mL (T75 flask). Spray the flask with 70% ethanol. Incubate the cells at 37°C at 5% CO₂. The cells should only be used until passage 35 at which point a fresh vial should be thawed.

HMC3 media change may be done as follows. Change growth media every 3 days unless the media changes colour. Pre-warm the HMC3 complete medium in a water bath set to 37°C for 30 minutes. Spray gloves with 70 % ethanol and transfer the cells from the incubator to a clean Class II safety cabinet. Collect the spent medium from flask and discard into a container containing chemgene. Gently pipette 5 mL (T25 flask) or 15 mL of HMC3 complete medium (T75 flask), taking care not to pipette directly onto the cells. Spray flasks with 70 % ethanol. Incubate cells at 37°C at 5 % CO₂.

HMC3 stocks may be made as follows. Pre-warm the PBS, trypsin-EDTA solution and HMC3 complete medium in a water bath set to 37°C for 30 minutes. HMC3 complete medium with 5% DMSO (HMC3 freezing medium) should be kept at 2-8°C. Spray gloves with 70 % ethanol, and transfer cells from the incubator to a clean Class II safety cabinet. Passage cells as in the passage protocol previously described. Transfer the required volume of cells into a 15 mL centrifuge tube. Centrifuge cells at 125 × g for 5 minutes at room temperature. Prepare 1 mL of HMC3 freezing media for every 1×10⁶ cells. Cover the tube containing the freezing media with aluminium foil and store at 4°C. Discard the supernatant into a beaker containing Virkon. Resuspend the cells in the required amount of freezing medium (1 mL for every 1×10⁶ cells). Aliquot 1 mL of the cell suspension into the required number of cryovials. Transfer the cryovials to freezing container and store at - 80°C for 24 h. Transfer vials to liquid nitrogen for long term storage.

### Rat hippocampal cells

The following solutions can be prepared: borate buffer or dH₂O depending on seeding surface (5ml) + poly-I-lysine (50 µL); 1X HBS: 45 mL MilliQ H₂O, 5 mL HBS (10X), 0.2255 g D-glucose; protease XIV: 4 mg in 4 mL HBS; borate buffer (100 mL, pH made to 8.5): 0.31 g boric acid, 0.475 g sodium tetraborate, 100 mL H₂O/distilled water.

To prepare plates, the following method may be used. Sterilise all equipment in ethanol. Wash each coverslip in 100% ethanol and then autoclaved water to remove residue, then leave for 1 hour. Dry the coverslips on ethanol sterilised blue paper and then place a in 24-well plate. Prepare the poly-I-lysine (10µg/mL), protease, and HBS solutions. Prepare the plating medium in 15-20 mL tubes and then place in an incubator (37°C at 5% CO₂). If using over two days, put them into the fridge to preserve the pH better. Use 500 µL for one 24-well plate well, 200 µL for 96-well plate well, and 1 mL for a 35 mm petri dish. Using a filter and a syringe, move the media mix into new tube. The plating medium only needs ~50 µL per coverslip/ well.

For 10 mL medium: 8.6 mL Neurobasal Medium, 1 mL FBS, 200 µL B27, 100 µL L-glutamine (200mM), 100 µL Pen-strep, and 0.33 µL FGF may be used. Using a syringe and filter, add drops (2-3, maximum of 5) to the coverslip/well centre. Hold and be careful when ejecting solution. Ensure the coverslips are not touching the well walls as this may cause the solution to run off. All/most of the coverslip/well should be coated. Leave in an incubator for 3 hours or overnight (dH2O poly-I-lysine only needs 1-2 hours). Remove the plates from the incubator and wash each 3-4 times with dH₂O. Filter drops onto the cover slip. Make sure the borate has been removed from filter before washing. Keep the plate open for the wells to dry out.

For dissection, the following method may be used. Sterilise all equipment and filter papers in ethanol (1/8th pieces, 2 per animal - used for dissecting on). Spray blue paper liberally with ethanol and dry in a fume hood. Filter the solutions into 10mm plates and place them on ice. During this method, the protease should be kept at room temperature. Dissect out the brain and place on new filter paper. Split the hemispheres, and fold over tissue to expose hippocampus and dissect out. Clean excess tissue while the tissue is on the small spatula. Once all the non-hippocampal tissue is removed, place in the HBS solution, then transfer to a second HBS dish. Once the dissection is complete, place the hippocampi into the protease solution and mash by cutting with scalpel into small chunks. Transfer the brains to a sterelin tube. Tissue should be stored in protease for 30 minutes (no more, or the cells may die) before removal. Place the tube into an incubator for at least 15 minutes.

Rat hippocampal cells may be plated and counted as follows. Place 1.5 mL of filtered HBS into 3 falcon tubes. Using a glass Pasteur pipette (largest bore), take the brains and place them into the first falcon tube, taking as little protease solution as possible. Gently shake to wash, let the tissue settle and then transfer to the second tube, again taking as little HBS as possible. Triturate ≥10x (with larger bore), then let the solution settle slightly before transferring any tissue that hasn't gone into suspension into the third tube. Triturate again ≥10x (smaller glass bore). If there are still big chunks present, triturate again. If the tissue/cell clumps don't disappear, it is best to exclude them (usually, 2-3 triturations per bore size is enough). Centrifuge tubes 2 & 3 for 3 minutes at 1400rpm. Remove the supernatant and re-suspend in 1 mL of culture medium (filtered twice) then combine the two solutions. Centrifuge this tube for 2 minutes at 1300rpm. Again, the supernatant is discarded, and the pellet re-suspended in ≥500 µL media, depending on pellet size. At this point, remove 15 µL of cell suspension and add to 15 µL of trypan blue solution (1:2 dilution). Place 10 µL of counting solution onto a haemocytometer and count the cells in each of the outer 4 quadrants and the middle quadrant. Calculatethe cell number by the following formula: (total amount of cells/ number of quadrants)*volume of cell suspension (mL)*dilution factor*10,000 (the dilution factor will be 2 in this case). Divide cells/mL by the plating cell density desired (e.g., 75,000/plate) and use this number to calculate volume of cells to plate onto each coverslip. Aim to use 50 µL of x cells per well, and if this is not possible then dilute more. Leave the plate in the incubator (37°C at 5% CO₂) for 1.5-2 hours before gently applying maintenance medium to each dish. The volume added depends on the size of vessel (50-100 µL for 96-well plate, 200-250 µL for coverslip in 24-well plate etc.). Return the cells to the incubator. Replace the culture media on day 3. Ideally, cells should be left until day 3 before checking. Medium should be changed at day 3 and then every 3-5 days, removing 75-90% of media, or all if required, depending on condition and rate of evaporation.

### Example 8: DNA damage-induced senescence in NPCs

Senescence may be induced by DNA damage and may serve as a valuable model to measure the therapeutic effects of the EVs of the invention.

In one exemplary method, DNA damage may be induced in neural progenitor cells (NPCs) with the DNA damage-inducer, doxorubicin (Figure 7).

In this protocol, NPCs were grown according to Example 7. The number cells needed for each assay was calculated, with plating density optimised based on the size of the well-used for the assays. The NPCs were then plated based on the optimised cell density and incubated for 48 hrs at 37°C in 5% CO₂.

After incubation, the cell media was aspirated, and fresh complete media added to the untreated cells while complete media containing 25 nM doxorubicin was added to the treated cells. Doxorubicin concentration and length of incubation may be determined by optimisation studies on NPCs.

The untreated and treated cells were incubated for 24 hrs at 37°C in 5% CO₂. After 24 hrs of incubation, the cell media was aspirated, and fresh complete media added to the untreated and treated cells. The cells were further incubated for 3 days at 37°C in 5% CO₂.

At the end of incubation, the cells were subjected to various techniques to establish the induction of senescence via DNA damage.

### Example 9: DNA damage-induced senescence in human microglial cell line HMC3

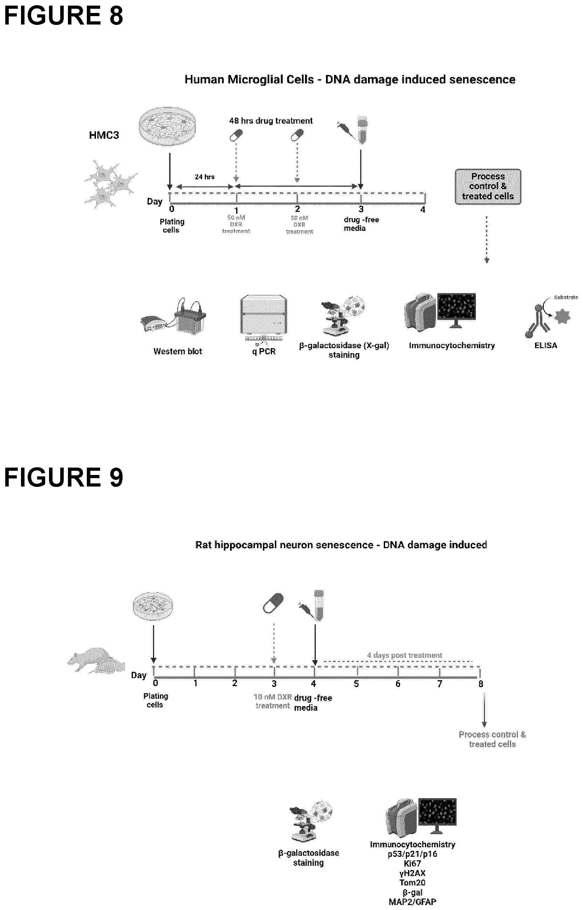

In another exemplary method, DNA damage may be induced in human microglial cells (HMC3 cells) with the DNA damage-inducer, doxorubicin (Figure 8).

In this protocol, HMC3 cells were grown according to Example 7. The number cells needed for each assay was then calculated, with plating density optimised based on the size of the well-used for the assays. HMC3 cells were plated based on the optimised plating density and incubated for 24 hrs at 37°C in 5% CO₂.

After incubation, the cell media was aspirated, and fresh complete media added to the untreated cells while complete media containing 50 nM doxorubicin was added to the treated cells. Doxorubicin concentration and length of incubation may be determined by optimisation studies on HMC3 cells.

Untreated and treated cells were incubated for 24 hrs at 37°C in 5% CO₂. After 24 hrs of incubation, the cell media was aspirated, and fresh complete media was added to the untreated cells while a second addition of complete media containing 50 nM doxorubicin was made to the treated cells. The cells were incubated for further 24 hrs at 37°C in 5% CO₂.

After incubation, the cell media was aspirated, and fresh complete medium added to the untreated and treated cells. The cells were then further incubated for 2 days at 37°C in 5% CO₂.

At the end of incubation, the cells were subjected to various techniques to establish the induction of senescence via DNA damage.

### Example 10: DNA damage-induced senescence in rat hippocampal cell culture

In another exemplary method, DNA damage may be induced in rat hippocampal cell culture with the DNA damage-inducer, doxorubicin (Figure 9).

In this protocol, rat hippocampal cells were first cultured according to Example 7. The cells were then plated on to a 13mm glass coverslips placed in the 24 well plate. The cells were incubated for 3 days at 37°C in 5% CO₂.

After 3 days of incubation, the cell media was aspirated, and fresh complete media added to the untreated cells while complete media containing 10 nM doxorubicin was added to the treated cells. Doxorubicin concentration and length of incubation may be determined by optimisation studies on rat hippocampal cells.

The untreated and treated cells were incubated for 24 hrs at 37°C in 5% CO₂. After 24 hrs of incubation, the cell media was aspirated, then fresh complete media was added to the untreated and treated cells. The cells were then incubated for further 4 days at 37°C in 5% CO₂.

At the end of incubation, the cells were subjected to immunocytochemistry (staining for various markers) to establish the induction of senescence via DNA damage.

### Example 11: Senescent markers shown in DNA damage-induced senescence models

Following the protocols in Examples 8-10, the cells were subjected to various techniques to establish the induction of senescence via DNA damage. A summary of the various senescence markers identified is shown in Table 3.

**Table 3: Summary of senescence hallmarks identified in treated cells**

| **Senescent cell hallmarks** | **Marker** | **Human Neural Progenitor Cells (NPCs)** | **Human Microglial Clone-3 cells (HMC3)** | **Rat Hippocampal culture (Neurons & Astrocytes)** |
|---|---|---|---|---|
| **DNA damage** | | | | |
| **Inducer concentration** | | Doxorubicin 25nM, 24 h | Doxorubicin 50nM, 48h | Doxorubicin 10nM, 24h |

| **Structural changes** | | | | |
|---|---|---|---|---|
| Morphology & cell size | Enlarged, flattened | ✔ | ✔ | |
| Increased lysosomal activity | SA-β-gal | ✔ | ✔ | ✔ |
| DNA damage | γ H2AX | ✔ | ✔ | ✔ |

| **Cell Cycle Arrest markers** | | | | |
|---|---|---|---|---|
| Lack of DNA synthesis | Cell cycle analysis | | | |
| Lack of proliferation | Ki67 | ✔ | ✔ | |
| Activation of p53-p21 pathway | ↑ p21, ↑ p53 | ✔ | ✔ | ✔ |
| Activation of p16-pRb pathway | ↑ p16 | ✔ | ✔ | ✔ |

| **Senescence Associated Secretory Phenotype (SASP)** | | | | |
|---|---|---|---|---|
| Enhanced SASP profile | ↑ IL-6 (ELISA) | ✔ | ✔ | |

## Claims

1. An isolated extracellular vesicle derived from a genetically modified cell, wherein the cell is engineered to express at least two exogenous genes encoding at least two proteins of interest or to overexpress at least two native genes encoding at least two proteins of interest, wherein the at least two proteins of interest comprise PGC-1α and PGC-1β.

2. The isolated extracellular vesicle of claim 1, wherein the proteins of interest further include one or more proteins selected from the group comprising: PPAR, NRF2, NRF1, TFAM, DRP1, OPAC1, SIRT3, POLG, TOP1MT, MRPL12, MRPS16, SIRT1, AMPK, FOXO3, KLOTHO, NeuroD1, BDNF, REST, and TERT, preferably wherein the further protein of interest is TERT, PPAR, NRF1 or NRF2, preferably wherein the PPAR protein is PPARγ.

3. The isolated extracellular vesicle of claim 1 or 2, wherein the extracellular versicles are selected from small extracellular vesicles or microvesicles.

4. The isolated extracellular vesicle of any preceding claim, wherein the extracellular vesicle comprises an increased density of whole mitochondria or mitochondrial biomolecules compared to that of an extracellular vesicle produced from an equivalent cell, wherein the equivalent cell has not been engineered to express the at least two exogenous genes at of interest, or wherein the equivalent cell has not been engineered to overexpress the at least two native genes of interest, and preferably wherein the density of whole mitochondria or mitochondrial biomolecules in the isolated extracellular vesicle has increased by at least 10%, at least 20%, at least 30%, at least 40%, at least 50%, at least 60%, at least 70%, at least 80%, at least 90%, at least 100%, at least 125%, at least 150%, at least 175%, or at least 200% compared to that of an extracellular vesicle produced from the equivalent cell.

5. The isolated extracellular vesicle of claim 4, wherein the mitochondrial biomolecules are mitochondrial DNA, mitochondrial RNA, mitochondrial proteins, mitochondrial lipids or mitochondrial polysaccharides, or mitochondrial peptides.

6. A method of preparing an isolated extracellular vesicle, the method comprising:
(a) subjecting a cell to genetic modification such that the cell is engineered to express at least two exogenous genes encoding at least two proteins of interest or to overexpress at least two native genes encoding at least two proteins of interest, wherein the at least two proteins of interest comprise PGC-1α and PGC-1β;
(b) culturing the cell, such that the extracellular vesicle is released from the cell; and
(c) isolating the extracellular vesicle released from the cell.

7. A genetically modified cell, wherein the cell is engineered to express at least two exogenous genes encoding at least two proteins of interest or to overexpress at least two native genes encoding at least two proteins of interest, wherein the at least two proteins of interest comprise PGC-1α and PGC-1β.

8. The isolated extracellular vesicle of any of claims 1 to 5, or the genetically modified cell of claim 7, wherein the genetically modified cell is a mesenchymal stem cell (MSC), hematopoietic stem cell (HSC), adipose-derived stem cell (ADSC), perivascular stem cell (PSC) or synovial stem cell (SSC); preferably wherein the genetically modified cell is selected from the group consisting of umbilical cord MSCs, umbilical cord blood MSCs, Wharton's jelly MSCs, placental MSCs, amniotic fluid MSCs, peripheral blood MSCs, bone marrow MSCs, adipocyte MSCs, dental pulp MSCs, synovial membrane MSCs, endometrium MSCs, skin MSCs, muscle MSCs, oral cavity MSCs, and periodontal ligament MSCs.

9. The genetically modified cell of claim 7 or 8, wherein the genetically modified cell comprises an increased density of whole mitochondria or mitochondrial biomolecules compared to that of an equivalent cell.

10. An *in vitro* or *ex vivo* method of increasing the density of whole mitochondria or mitochondrial biomolecules in a cell, the method comprising subjecting the cell to genetic modification such that the cell is engineered to express at least two exogenous genes encoding at least two proteins of interest or to overexpress at least two native genes encoding at least two proteins of interest, wherein the at least two proteins of interest comprise PGC-1α and PGC-1β.

11. A nucleic acid construct comprising a nucleic acid sequence comprising coding sequences for at least two genes which encode at least two proteins of interest, wherein the at least two proteins of interest comprise PGC-1α and PGC-1β.

12. The isolated extracellular vesicle of any one of claims 1 to 5 or claim 8, the method of claim 6 or claim 10, the genetically modified cell of any one of claims 7 to 9, or the nucleic acid construct of claim 11, wherein the proteins of interest further include one or more proteins selected from the group comprising: PPAR, NRF2, NRF1, TFAM, DRP1, OPAC1, SIRT3, POLG, TOP1MT, MRPL12, MRPS16, SIRT1, AMPK, FOXO3, KLOTHO, NeuroD1, BDNF, REST, and TERT, preferably wherein the further protein of interest is TERT, PPAR, NRF1 or NRF2, preferably wherein the PPAR protein is PPARγ.

13. A host cell, or a cell free expression system, comprising the nucleic acid construct of claim 11 or 12.

14. A pharmaceutical composition comprising the isolated extracellular vesicle of any one of claims 1 to 5, claim 8 or claim 12, the genetically modified cell of any one of claims 7 to 9 or claim 12, the nucleic acid construct of claim 11 or 12, or the host cell or the cell free expression system of claim 13, and a pharmaceutical acceptable carrier or excipient.

15. The isolated extracellular vesicle of any one of claims 1 to 5 claim 8 or claim 12, the genetically modified cell of any one of claims 7 to 9 or claim 12, the nucleic acid construct of claims 11 or 12, the host cell or the cell free expression system of claim 13, or the pharmaceutical composition of claim 14, for use in medicine, preferably wherein isolated extracellular vesicle, the genetically modified cell, the nucleic acid construct, the host cell, the cell free expression system, or the pharmaceutical composition is for use in a method of treating an age-related or neurodegenerative disease, more preferably wherein the neurodegenerative disease is selected from the group consisting of Alzheimer's disease, dementia, multiple sclerosis, amyotrophic lateral sclerosis, Parkinson's disease, Huntington's disease, multiple system atrophy, spinal muscular atrophy, cerebral palsy, Rett syndrome or epilepsy, or wherein the age-related disease is selected from the group consisting of cancer, cardiovascular diseases, metabolic disorders, joint disorders including osteoarthritis, ocular disorders, immune system disorders, digestive and gastrointestinal disorders or dermal disorders.
